# NEW EUROPEAN PATENT SPECIFICATION

(11) **EP 1 131 087 B2**
(45) Date of publication and mention of the opposition decision: **01.04.2009**
(45) Mention of the grant of the patent: 08.09.2004
(21) Application number: 99958892.4
(22) Date of filing: 12.11.1999
(51) Int. Cl.: A61K 38/18, A61P 35/00

(54) **ALLEVIATATION OF PROSTATE CANCER SYMPTOMS**
VERMINDERUNG VON PROSTATAKREBSSYMPTOMEN
SOULAGEMENT DES SYMPTOMES DU CANCER DE LA PROSTATE

(30) Priority: 13.11.1998 US 191239
(43) Date of publication of application: 12.09.2001
(60) Divisional application: 04008347.9
(73) Proprietor: Stryker Corporation, Kalamazoo, Michigan 49001 (US)
(72) Inventor: SAMPATH, Kuber, T., Holliston, MA 01746 (US); COHEN, Charles, M., Weston, MA 02193 (US); RUEGER, David, C., Southborough, MA 01772 (US)
(74) Representative: Lock, Graham James
(86) International application number: PCT/US1999/026636
(87) International publication number: WO 2000/029012

(56) References cited:
- EP-B1- 0 575 555
- EP-B1- 0 653 942
- EP-B1- 0 756 628
- WO-A-97/11095
- WO-A-98/50061
- US-A- 5 652 118
- US-A- 5 739 107
- ANDREWS PETER W ET AL: "Inhibition of proliferation and induction of differentiation of pluripotent human embryonal carcinoma cells by osteogenic protein-1 (or bone morphogenetic protein-7)." LABORATORY INVESTIGATION, vol. 71, no. 2, 1994, pages 243-251, XP000929102 ISSN: 0023-6837
- SODA HIROSHI ET AL: "Antiproliferative effects of recombinant human bone morphogenetic protein-2 on human tumor colony-forming units." ANTI-CANCER DRUGS, vol. 9, no. 4, April 1998 (1998-04), pages 327-331, XP000915431 ISSN: 0959-4973

## Description

### BACKGROUND OF THE INVENTION

Cancer is a major cause of death throughout the world. A characteristic feature of cancer is cell growth not regulated by external signals. Cancer cells also may develop the capacity to metastasize and to colonize distant sites. See generally HARRISON'S PRINCIPLES OF INTERNAL MEDICINE, 505 (Fauci *et al*. eds., 14th ed., 1998). Cancer is most common in tissue that has a relatively rapid cell turnover rate. In most cases, two types of cells exist in those tissue types: dividing progenitor cells and non-dividing differentiated cells. The term cancer refers to both benign growths in which cells divide without regulation, but are unable to metastasize, and malignant growths in which cells divide without regulation, and are able to metastasize.

In the absence of a disease, such as cancer, or trauma, once a cell has acquired a particular differentiated state, its fate is typically irreversible. Morphogens are known to induce the proliferation and differentiation of progenitor cells in numerous tissues. Morphogens include members of the family of bone morphogenetic proteins (BMPs) identified by their ability to induce ectopic, endochondral bone morphogenesis. The morphogens, also referred to as osteogenic proteins, generally are classified as a subgroup of the TGF-β superfamily of growth factors. Hogan, Genes & Development 10: 1580-1594 (1996). Members of that superfamily include the mammalian osteogenic protein-1 (OP-1, also known as BMP-7, and the *Drosophila* homolog 60A), osteogenic protein-2 (OP-2, also known as BMP-8), osteogenic protein-3 (OP-3), BMP-2 (also known as BMP-2A or CBMP-2A, and the *Drosophila* homolog DPP), BMP-3, BMP-4 (also known as BMP-2B or CBMP-2B), BMP-5, BMP-6 and its murine homolog Vgr-1, BMP-9, BMP-10, BMP-11, BMP-12, GDF3 (also known as Vgr2), GDF8, GDF9, GDF10, GDF11, GDF12, BMP-13, BMP-14, BMP-15, GDF-5 (also known as CDMP-1 or MP52), GDF-6 (also known as CDMP-2), GDF-7 (also known as CDMP-3), the Xenopus homolog Vgl and NODAL, UNIVIN, SCREW, ADMP, and NEURAL.

Morphogens share common structural features. The mature form results from processing through a "pro-form" to yield a mature polypeptide containing a carboxyl terminal active domain of approximately 97-106 amino acids. All morphogens share a conserved pattern of cysteines in that domain. The active form is either a disulfide-bonded bornodimer of a single superfamily member or a heterodimer of two different members. *See*, *e*.*g*., Massague, Annu. Rev. Cell Biol. 6: 597 (1990); Sampath et al., J. Biol. Chem. 265: 13198 (1990).

Efforts to alleviate the symptoms of cancer, totally or partially, have often focused on physical excision of cancerous tissue, radiation-induced destruction of cancerous tissue, and chemical-induced destruction of cancerous tissue. While these modes of treatment have given hope to those afflicted with cancer, they have drawbacks in many instances including invasiveness (in the case of physical excision), cytotoxicity, production of serious side effects in the treated subject, limited therapeutic windows, and limited ability to completely destroy a cancerous tissue. Additionally, some less traditional efforts have been made to develop biological agents that regulate the host's own biological machinery to respond to cancer cells. Moreover, not all anti-cancer agents are equally efficacious on all cell types. Thus, a need exists for anti-cancer agents that are capable of alleviating the symptoms of breast or prostate cancer and have fewer problems associated with their use than current anti-cancer agents.

### SUMMARY OF THE INVENTION

The present invention provides use of a morphogen as defined in the claims of the present application. Many cancers are characterized by cells that display unregulated growth with or without the ability to metastasize to additional sites throughout the body. One therapeutic use of the invention to alleviate the symptoms of breast or prostate cancer is to inhibit the unregulated proliferation of cancerous cells. Morphogens (also known as bone morphogenic proteins, "BMPs," or "morphogenic proteins") are a family of proteins capable of altering cellular phenotype in many different cell types. In particular; morphogens are capable of stimulating, maintaining, or regenerating a differentiated cell phenotype. It has now been discovered that exposing breast cancer cells to the morphogen OP1 or prostate cancer cells to OP1 or BMP6 inhibits cancer cell growth and causes such cells to differentiate away from the cancerous phenotype. Thus, use of these morphogens can influence cancer cell fate and, in turn, alleviate the symptoms of breast or prostate cancer.

The present invention is an alternative approach to methods currently being used to treat cancer and alleviate symptoms of this disease. Therapeutic uses of the invention also reduce the side effects typically observed with conventional chemotherapy methods for treating breast or prostate cancer.

Morphogens are dimeric proteins having an amino acid sequence of either at least 70% homology with the C-terminal seven-cysteine skeleton of human OP-1 which includes residues 330-431 of SEQ ID NO: 2, or an amino acid sequence having at least 60% identity with this C-terminal seven-cysteine skeleton of human OP-1, SEQ ID NO: 3, SEQ ID NO: 4, SEQ ID NO: 5, SEQ ID NO: 6, or SEQ ID NO: 7, which can inhibit the growth of cancer cells. Morphogens useful in the invention are OP-1 and BMP-6 with regard to prostate cancer or OP1 with regard to breast cancer.

In another aspect of the invention, the morphogen is formulated with a pharmaceutically acceptable carrier or vehicle. The carrier or vehicle may be a physiologically acceptable aqueous solution such as a saline or buffer solution (*e*.*g*., a phosphate buffered saline solution). The carrier or vehicle comprises a liposome or microsphere that is biocompatible, biodegradable and/or bioerodable. Alternatively, the carrier or vehicle comprises a biocompatible matrix, which is biodegradable or bioerodable. Compounds and substances useful as matrices in the invention include, without limitation, proteinaceous materials, (*e*.*g*., collagen), mono- and polysaccharides and derivatives thereof, (*e*.*g*., carboxymethyl cellulose and hyaluronic acid), synthetic polymers (*e*.*g*., polyglycolic and polylactic acid polymers or co-polymers), calcium-containing compounds, (*e*.*g*., hydroxyapatite or tricalcium phosphate), and combinations thereof.

In one aspect of the invention, the morphogen OP1 may be delivered to a breast cancer cell or the morphogen OP1 or BMP6 may be delivered to a prostate cancer cell in a cell that is capable of expressing the morphogen. In another aspect of the invention, the morphogen may be delivered as a non-infectious, non-integrating DNA encoding said morphogen that is operably linked to a promoter which directs expression of the morphogen in cells of a particular tissue. In another aspect of the invention, an enhancer element is in operative association with the promoter. In yet another embodiment, DNA encoding a morphogen is operably linked to a promoter. The DNA is associated with a liposome, microsphere, or viral particle that facilitates uptake of the DNA and expression of the morphogen by cancer cells or by neighboring non-cancer cells. Viral particles useful in transducing DNA encoding morphogen into cancer cells or neighboring non-cancer cells include adeno-associated virus particles.

In a preferred embodiment, symptoms of cancer are alleviated in breast cancer, by OP1 or prostate cancer (androgen-dependent and -independent).

In a preferred embodiment of the invention, by OP1 or BMP6, the cancer is treated by the therapeutic use of the morphogen to inhibit the unregulated proliferation of the cancer cells or to stimulate differentiation of the cancer cells away from the cancer phenotype. In another aspect of the invention, morphogens are used to treat cancer to alleviate other symptoms ofcancer comprising abnormal bleeding, abnormal bodily function, abnormal cell morphology, abnormal enzyme levels, abnormal hormone levels, abnormal oncofetal antigen levels, abnormal tissue growth, abnormal tissue mass, abnormal tumor-associated protein levels, altered neurologic function, altered neurologic structure, angiogenesis, diarrhea, effusions, fatigue, fever, lesions, malnutrition, metastasis, nausea, obstruction of a bodily passageway, opportunistic infection, pain, poor Kamofsky performance status, presence of cell-surface markers, presence of histological cancer markers, presence of intracellular markers, presence of molecular markers, tumor invasion, urinary frequency, weight loss, and combinations thereof. Inhibition of proliferation may be measured, for example, by increased survival rates, decreased tumor size, or tumor regression.

The expression of bone morphogenic protein receptors (BMPRs) on targeted cancer cells provides a means for determining tissue likely to respond to morphogen treatment. In a preferred embodiment, targeted malignant cells in a patient are pre-screened for expression of BMPRs to enable the selection of patients with a high probability of responding to a given BMP treatment.

Morphogens inhibit cellular growth as measured, for example, by cellular incorporation of [³H]-thymidine in an *in vitro* assay, shrinkage in tumor size, or decrease in expression of cancer-associated antigens. In another aspect of the invention, morphogen stimulates cancer cells to differentiate away from the cancer phenotype, for example, as indicated by decreased expression of cancer-associated antigens, a change in cell morphology, or an increased expression of antigenic or biochemical markers associated with a preferred differentiated cell phenotype. In another aspect of this invention, morphogen may be applied to an affected tissue that has been removed from the host in an *ex vivo* protocol. In therapeutic uses of the invention, morphogen may be employed in combination with one or more non-morphogenic agents, such as known anti-cancer agents, analgesics, or anti-inflammatory compounds.

Another preferred embodiment of the invention comprises the therapeutic use defined in Claim 1 or 2, wherein the morphogen inhibits cellular incorporation of [³H]-thymidine in an *in vitro* assay.

The invention will be understood further from the following drawings, description, and claims.

### BRIEF DESCRIPTION OF THE DRAWINGS

FIGS. 1A-1M are tabular alignments of the amino acid sequences of various naturally occurring morphogens with a preferred reference sequence of human OP-1, residues 330-431 of SEQ ID NO: 2.

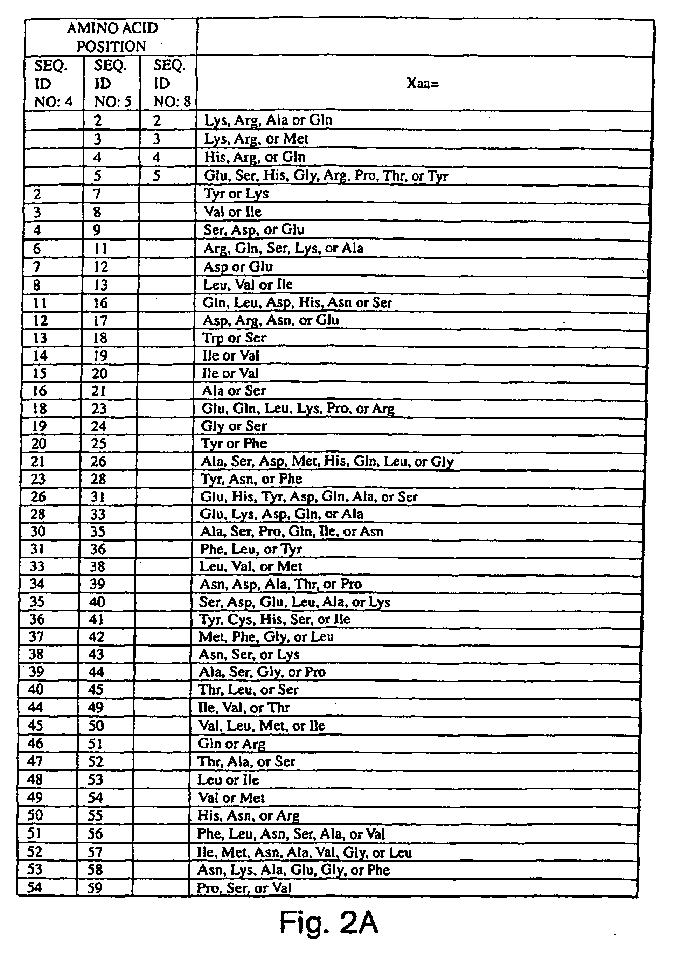

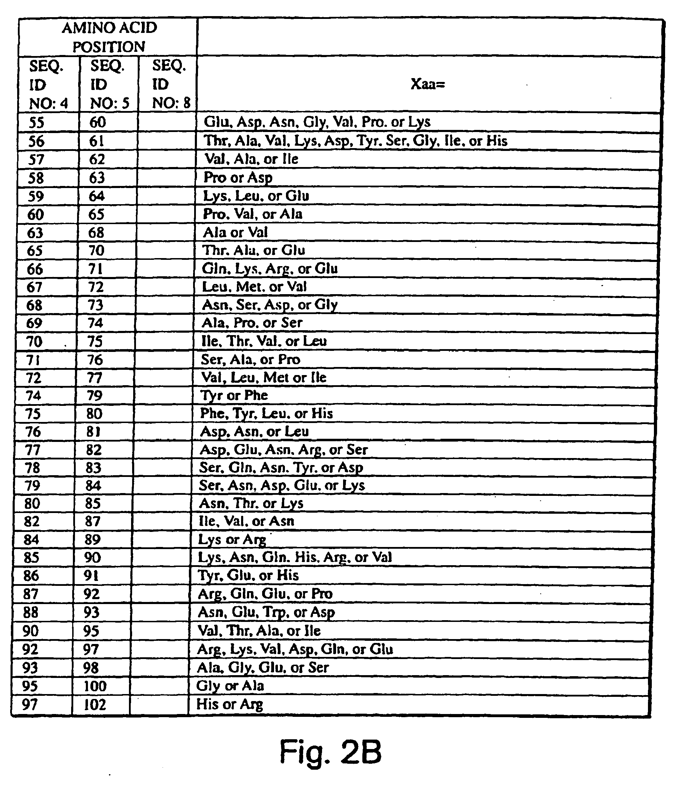

FIGS. 2A-2B are tabular presentations of alternative amino acids for "Xaa" positions in generic sequences SEQ ID NOS: 4, 5, and 8 that represent amino acid variations in known morphogens.

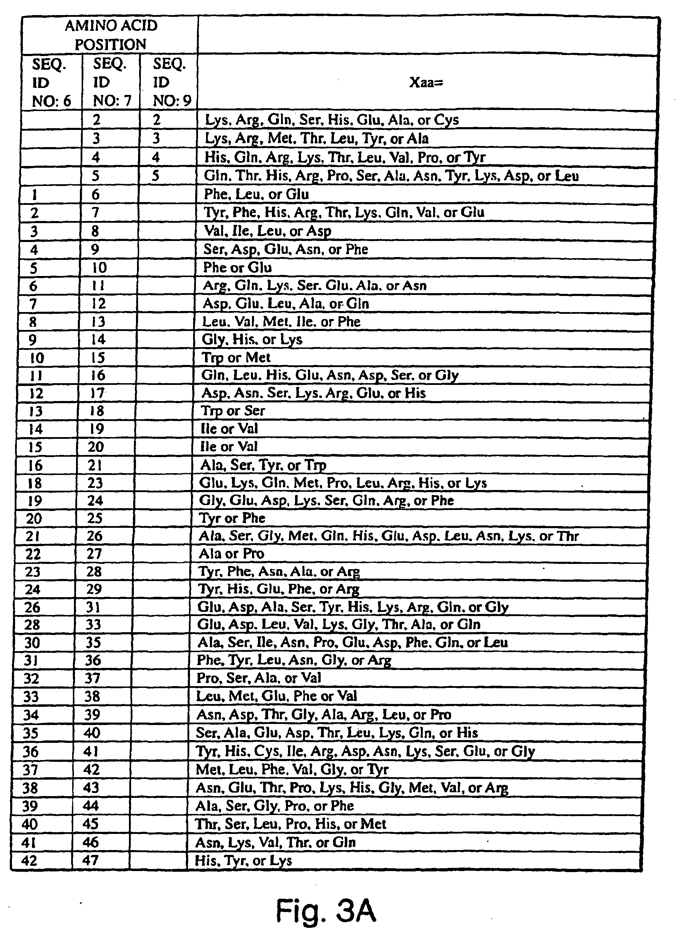

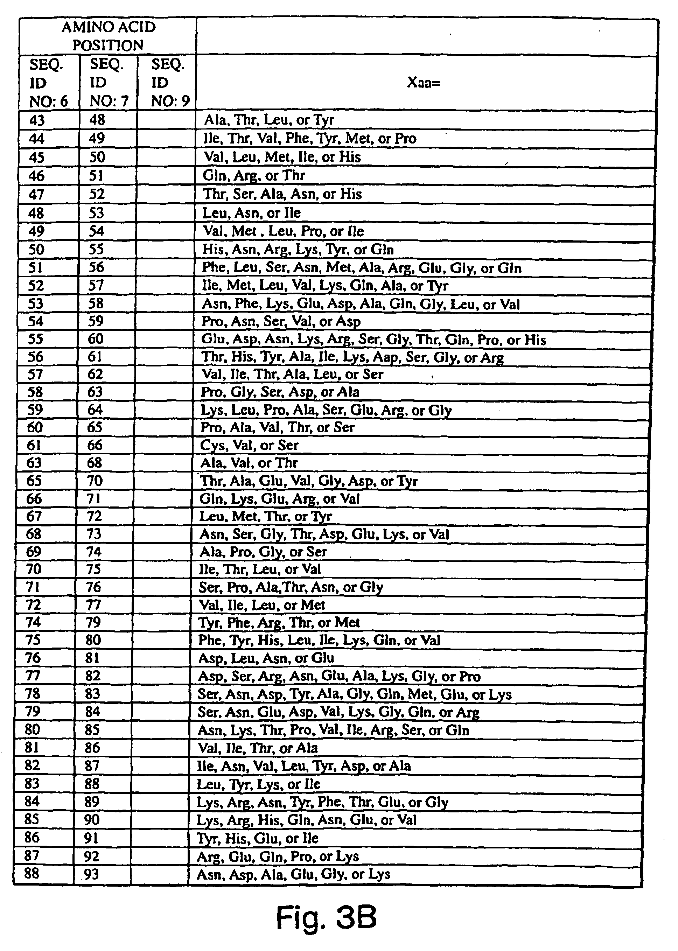

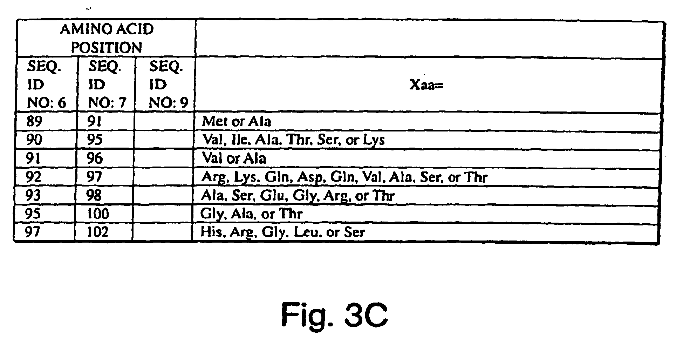

FIGS. 3A-3C are tabular presentations of alternative amino acids for "Xaa" positions in generic sequence SEQ ID NOS: 6, 7, and 9 that represent amino acid. variations in known morphogens.

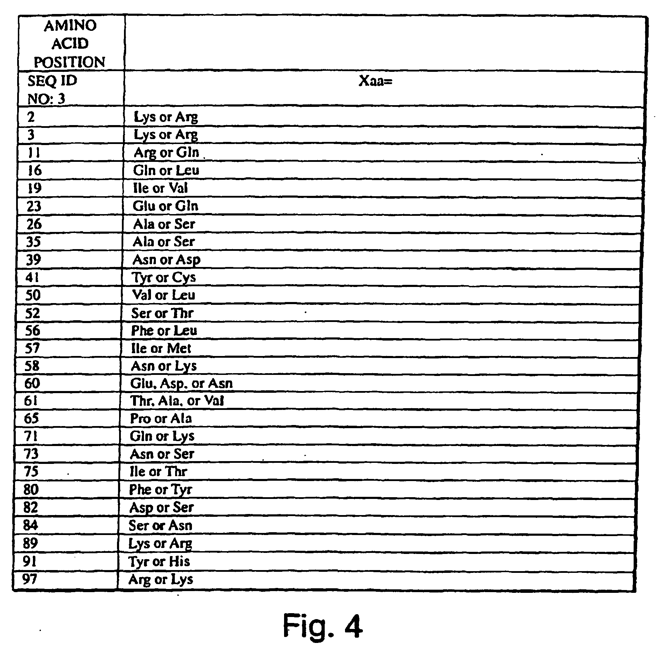

FIG. 4 is a tabular presentation of alternative amino acids for "Xaa" positions in generic sequence SEQ ID NO: 3 that represents amino acid variations in several identified allelic and phylogenetic variants of OP-1 and OP-2.
FIGS. 5A-5B illustrates the ability ofmorphogen (OP-1) to induce undifferentiated NG108 cells (FIG. 5A) to undergo differentiation of neural morphology (FIG. 5B).
FIGS. 6A-6D show the effect of morphogen (OP-1) on human embryo carcinoma cell redifferentiation.
FIG. 7 shows the effect ofmorphogen (BMP 6 or OP-1 (BMP 7)), Activin or TGF-β1 on cell growth of unstimulated androgen responsive prostate cancer LNCaP cells.
FIG. 8 shows the effect of morphogen (BMP-6 or OP-1 (BMP-7)), Activin or TGF-β1 on cell growth of stimulated androgen-responsive prostate cancer LNCaP cells.
FIG. 9 shows the effect of follistatin on the bioactivity of morphogens (BMP-6 or OP-1 (BMP-7)) or Activin in androgen-responsive prostate cancer LNCaP cells.
FIGS. 10A-10B show the effect of morphogen (OP-1) or TGF-β1 on cell growth of estrogen responsive human breast cancer MCF-7 cells in the absence (FIG. 10A) or in the presence (FIG. 10B) of 5% serum containing medium.
FIGS. 11A-11B show the effect ofmorphogen (OP-1) on cell growth of 17-β estradiol stimulated or unstimulated estrogen responsive human breast cancer MCF-7 cells (FIG. 11A) and the effect of TGF-β1 on cell growth of 17-β estradiol stimulated or unstimulated estrogen responsive human breast cancer MCF-7 cells (FIG. 11B).
FIG. 12 shows the effect ofmorphogen (OP-1), tamoxifen, or both morphogen (OP-1) and tamoxifen combined on cell growth of estrogen-responsive human breast cancer MCF-7 cells.
FIG. 13 shows the effect of morphogen (OP-1) on cell growth of human glioblastoma A-172 cells.
FIG. 14 shows the effect ofmorphogen (OP-1) or TGF-β2 on cell growth of human lung carcinoma A-549 cells.
FIG. 15 shows the expression of three BMPRs in murine prostate cancer cell lines E4 and F11.
FIG. 16 shows the effect of morphogen (BMP-6) on cell growth of murine prostate cancer cell lines E4 and F11.
FIG. 17 shows the effect of morphogen (BMP-6) on tumor volume in mice with prostate cancer xenografts.
FIG. 18 shows the effect of morphogen (BMP-6) on tumor volume in mice with prostate cancer xenografts, corrected for surviving animals.
FIGS. 19A-19B shows necrosis and diffuse inflammation in tumors treated with morphogen (BMP-6) as compared to control tumors.

### DETAILED DESCRIPTION OF THE INVENTION

The present invention provides the use of OP1 in the manufacture of a medicament for alleviating the symptoms of breast cancer or the use of OP1 or BMP6 in the manufacture of a medicament for alleviating the symptoms of prostate cancer. Many cancers are characterized by cells that display unregulated growth with or without the ability to metastasize to additional sites throughout the body. One therapeutic use of the invention to alleviate the symptoms of cancer is to stop cancerous cells from growing and dividing. The morphogens are capable of differentiating progenitor cells (*i.e.*, dividing cells). Exposure to the morphogens inhibits growth of the cancer cells. Thus, therapeutic use of the morphogens influences cancer cell fate and, in turn, alleviates various characteristic symptoms of the cancer.

BMP signal transduction follows the paradigm established by TGF-β signaling. As with TGF-β, BMPs signal through an interaction with a heteromeric complex of membrane receptors, type I and type II. Specifically, ligand binding results in cross-phosphorylation of type I receptor by type II receptor; type II receptor, in turn, propagates BMP signaling. *See,* Yamashita el al., Bone 19: 569-574 (1996). *In vitro* experiments have shown that all members of BMP that belong to the TGF-β superfamily bind to BMPR-II in combination with BMPR-IA or IB. *See,* Yamashita *et al*., *supra.* Thus, BMPR-IA , BMPR-IB, and BMPR-II appear to be BMP-specific receptors, and are likely to mediate morphogen inhibition of cancer cell growth.

### I. USEFUL MORPHOGENS

In its mature, native form, natural-sourced morphogen is a glycosylated dimer, typically having an apparent molecular weight of about 30-36 kDa as determined by SDS-PAGE. When reduced, the 30 kDa protein gives rise to two glycosylated peptide subunits having apparent molecular weights of about 16 kDa and 18 kDa. In the reduced state, the protein has no detectable osteogenic activity. The unglycosylated protein, which also has osteogenic activity, has an apparent molecular weight of about 27 kDa. When reduced, the 27 kDa protein gives rise to two unglycosylated polypeptide chains, having molecular weights of about 14 kDa to 16 kDa. Typically, the naturally occurring osteogenic proteins are translated as a precursor, having an N-terminal signal peptide sequence typically less than about 30 residues, followed by a "pro" domain that is cleaved to yield the mature C-terminal domain. The signal peptide is cleaved rapidly upon translation, at a cleavage site that can be predicted in a given sequence using the method of Von Heijne, Nucleic Acids Res. 14: 4683-4691 (1986). The pro-domain typically is about three times larger than the frilly processed mature C-terminal domain.

Morphogens useful in the methods of the invention are OP1 and BMP6 with regard to prostate cancer or OP1 with regard to breast cancer.

### II. FORMULATION AND TREATMENT CONSIDERATIONS

As a general matter, the therapeutic uses of the present invention may be applied to the treatment of any mammalian subject afflicted with breast or prostate cancer. One of ordinary skill in the medical or veterinary arts is trained to recognize whether a mammal is afflicted with the cancer. For example, routine testing and/or clinical or veterinary diagnostic evaluation will reveal whether the mammal has any of the symptoms of the cancer. Symptoms of the cancer include unregulated cell proliferation and/or proliferation of cells that have a cancer-cell phenotype. Other common symptoms of the cancer include abnormal bodily function, abnormal cell morphology, abnormal enzyme levels, abnormal hormone levels, abnormal oncofetal antigen levels, abnormal tissue growth, abnormal tissue mass, abnormal tumor-associated protein levels, altered neurologic function, altered neurologic structure, undesirable angiogenesis that provides a tumor with a blood supply, abnormal bleeding, diarrhea, effusions, fatigue, fever, lesions, malnutrition, metastasis, nausea, obstruction of a bodily passageway, opportunistic infection, pain, poor Kamofsky performance status, presence of cancer-associated cell-surface markers, presence of cancer-associated histological markers, presence of cancer-associated intracellular markers, presence of cancer-associated molecular markers, tumor invasion, urinary frequency, and weight loss. See generally, HARRISON'S PRINCIPLES OF INTERNAL MEDICINE 493-627 (Fauci *et al*. eds., 14th ed. 1998).

Cancer can affect any tissue in the body. However, as described above, some tissue sites are more likely to be affected with cancer. The cancers that compositions and methods of the present invention are anticipated to treat are breast cancer or prostate cancer. See generally, HARRISON'S PRINCIPLES OF INTERNAL MEDICINE 493-627 (Fauci *et al*. eds., 14th ed. 1998).

Cancer cell growth is typified by a general unresponsiveness to extracellular signals. However, in accordance with the present invention, it has now been discovered that the morphogen OP1 can regulate breast cancer cell growth and the morphogens OP1 or BMP6 can regulate prostate cancer cell growth. As discussed above, BMPRs mediate BMP signaling. For example, in prostate cancer, it has been shown that BMP-2 decreases the rate of proliferation ofLNCaP cells (*see* ten Dikje et al., J. Biol. Chem. 269: 16985-988 (1994)). It has also been demonstrated that the prostate expresses very high levels of BMPR-IB (*see* Ide et al., Oncogene 14:1377-382 (1997); Autzen et al., Br. J. Cancer 78: 1219-223 (1998)). In accordance with the present invention, it has now been shown that BMP-6 inhibits the growth of murine prostate tumor xenografts expressing all three types of BMPRs (*see* Example 7, *infra*). Hence, the determination of BMPRs expression on targeted malignant cells represents a novel means to pre-screen a subject to enable the selection of subjects with high probability of responding to a given morphogen. Targeted prostate or breast cells may be obtained from a patient, and the expression of specific BMPRs determined, for example, by messenger RNA isolation and Northern Blot analysis. An appropriate morphogen may then be selected from OP1 or BMP6 for prostate or from OP1 for breast which is most likely to provide an effective treatment based upon the BMPR expression in the targeted cells. Alternatively, patients with a low probability or responding to morphogen treatment based upon the absence of requisite BMPR expression on targeted tissue may be referred for alternative therapies.

In use of the present invention the morphogens or the morphogens combined with one or more other agents in the present invention may be suitable for administration by any compatible route. For example, the morphogens may be suitable for direct (*e*.*g*., locally, as by injection, implantation or topical administration to a tissue locus), systemic (*e.g.*, parenterally or orally), external (*e.g.*, an i.v. bag), or internal (*e*.*g*., a bioerodable implant, or a colony of implanted, morphogen-producing cells) delivery and, once delivered, are capable of inhibiting cancer cell proliferation and/or stimulating differentiation of cells having a cancer cell phenotype. In a preferred embodiment, following direct injection of morphogen into the tumor tissue, the morphogen results in necrosis of the tumor and diffuse inflammation. The agent preferably comprises part of an aqueous or physiologically compatible fluid suspension or solution for parenteral administration such as intravenous, subcutaneous, intramolecular, ophthalmic, intraperitoneal, intramuscular, buccal, rectal, vaginal, intraorbital, intracerebral, intracranial, intraspinal, intraventricular, intrathecal, intracisternal, intracapsular, intranasal, or aerosol administration. The morphogen carrier or vehicle is physiologically acceptable so that in addition to delivery of the desired agent to the patient, it does not otherwise adversely affect the patient's electrolyte and/or volume balance. The fluid medium for the agent thus can comprise normal physiologic saline (*e*.*g*., 9.85% aqueous NaCl, 0.15M, pH 7-7.4). Those skilled in the art can prepare useful solutions for any type of parenteral administration as described, for example, in REMINGTON'S PHARMACEUTICAL SCIENCES (Gennaro, A., ed., Mack Pub., 1990). Dispersing the morphogen, for example, with a dermatologically acceptable carrier such as a lotion, cream, ointment or soap is an appropriate formulation for topical administration to the skin surface and is particularly useful for treating skin cancer.

Association of the mature morphogen dimer with a morphogen pro-domain results in the pro-form of the morphogen that typically is more soluble in physiological solutions than the corresponding mature form. In fact, endogenous morphogens are thought to be transported (*e*.*g*., secreted and circulated) in the mammalian body in this form. Culture medium of morphogen-secreting mammalian cells (*e*.*g*., cells that are transfected with nucleic acid encoding the morphogen and that are competent to express the morphogen) is one source of this soluble form of the protein. Alternatively, complexing the mature, morphogenically-active polypeptide dimer (or an active fragment thereof) with a morphogen pro-domain polypeptide or a solubility-enhancing fragment thereof creates a soluble species. Solubility-enhancing pro-domain fragments can be any N-terminal, C-terminal, or internal fragment of the pro region of a member of the morphogen family that complexes with the mature polypeptide dimer to enhance stability and/or dissolubility of the resulting noncovalent or covalent complex. Typically, those fragments cleaved at the proteolytic site Arg-Xaa-Xaa-Arg are useful. A detailed description of soluble complex forms of morphogenic proteins, including how to make, test and use them, is described in WO 94/03600 (PCT US 93/07189). In the case of OP-1, useful pro-domain polypeptide fragments include the intact pro-domain polypeptide (residues 3 0-292) and fragments 48-292 and 15 8-292, all of SEQ ID NO: 2. Additionally, other molecules enhance solubility and are particularly useful for oral administrations. For example, addition of 0.2%casein increases solubility of the mature active form of OP-1 by 80%. Other components found in milk and/or various serum proteins may also be useful.

In use of the present invention, the morphogens may be suitable for oral administration. In contrast, oral administration of proteins generally is unsuccessful, because digestive enzymes and acids in the mammalian digestive system degrade most proteins before they are absorbed into the bloodstream. However, unlike most proteins, morphogens are typically acid stable and protease-resistant. See, *e*.*g*., U.S. Patent No. 4,968,590. In addition, mammary gland extract, colostrum, and 57-day milk contain at least one morphogen, OP-1. The OP-1 purified from mammary gland extract is morphogenically-active and is detected in the bloodstream. Thus, maternal administration, via ingested milk, may be a natural delivery route of OP-1 and other morphogens proteins. See *e.g.,* Letterio et al., Science 264: 1936-1938 (1994). Moreover, the morphogen found in milk (and mammary gland extract and colostrum) is readily soluble, probably by association with a pro-domain and/or another milk component.

Alternatively or in addition, a delivery vehicle competent for transport through the gastrointestinal system is useful for oral administration of morphogens in accordance with the present invention. For example, the morphogen can be a part of a biologically credible microsphere that interacts with the gastrointestinal lining and absorb through this lining. Useful polymers include, for example, polystyrene, polyactide and/or polyglycolide and/or polycyanoacrylate polymers and combinations thereof.

Those skilled in the art can modify morphogens of the present invention to be derivatized or conjugated to a lipophilic moiety or to a substance that is actively transported across the blood-brain barrier. See, *e*.*g*., Pardridge, Endocrine Reviews 7:314-330 (1986) and U.S. Patent No. 4,801,575.

Association of molecules capable of targeting morphogens to a desired tissue with morphogens of the present invention is possible. For example, an antibody, antibody fragment, or other binding protein that interacts specifically with a surface molecule on cells of the desired tissue, is appropriate to target morphogens. The single chain binding site technology disclosed in U.S. Patent No. 5,091,513 is useful to design targeting molecules.

*In vitro*, *in vivo,* or *ex vivo* applications are useful to expose affected tissues to morphogens. Morphogens for use in the present invention, as well as cells capable of expressing said morphogens, are suitable for delivery to the affected tissue. Those skilled in the art can construct such cells capable of expressing morphogens using standard means. Biocompatible, biodegradable, bioerodable matrices such as collagen or hydroxyapatite are useful to permit cells to infiltrate and grow into the matrix material during differentiation. Moreover, one may expose affected tissues that have been removed from the host, and expose them to any of the variety of morphogens described herein. The treated tissues are than suitable for return to the host. Any of these *in vitro*, *in vivo*, or *ex vivo* applications are useful to inhibit the cancer cell proliferation and/or stimulate differentiation of cells having the cancer cell phenotype away from the cancer cell phenotype. In one aspect of the invention, the growth of LNCaP cells, MCF-7 cells is inhibited.

As will be appreciated by one of ordinary skill in the art, the formulated compositions contain therapeutically effective amounts of the morphogen. The effective dose and dosage strategy will vary depending upon a number of factors including the biological efficacy of the selected agent, the chemical characteristics (*e*.*g*., hydrophobicity) of the specific agent, the formulation of the agent, the administration route, the target location, the pharmacokinetics of the agent, the condition of the diseased tissue, and the overall health status of the particular mammal. For example, intratumoral administration of about 200 µL of 100 ng/mL BMP-6 inhibited tumor growth in mice (*see* Example 7, *infra*).

Morphogens of the invention may be employed alone or in combination with other molecules known to be beneficial in the treatment of cancer. For example, one can employ various well-known anti-tumor agents, analgesics, or anti-inflammatory compounds with a morphogen. Additionally, one can employ various well-known growth factors, hormones, enzymes, therapeutic compositions, antibiotics, or other bioactive agents with a morphogen.

### III. EXAMPLES

The following examples provide experimental evidence of the effect of various morphogens on cell differentiation and cell growth in various cell types. Each of these cancer cell lines is characterized by some form of unregulated growth. As shown below in a variety of examples, the cell growth of the cancer cells exposed to the morphogens was inhibited. Moreover, in several of the examples, morphological characteristics of cell differentiation were observed. These experimental data provide positive evidence that OP1 can alleviate the symptoms of breast cancer and OP1 or BMP6 can alleviate the symptoms of prostate cancer, possibly by inhibiting cell growth and/or stimulating cell differentiation of the cancer cells.

### Example 1 Morphogen Inhibition of Proliferation of the Androgen-Responsive Prostate Cancer Cell Line LNCaP

Approximately 25% of all males over the age of 55 years old are afflicted by some form of prostate disease. Androgens are known to stimulate prostate cancer cell proliferation. Typically, prostate cancer cells eventually lose their dependence on androgen to proliferate and the cancer progresses to a more malignant, androgen-independent state. Loss of androgen responsiveness is also a characteristic of metastatic forms of prostate cancer. Thus, a common method of treating prostate cancer is to employ some form of androgen control or ablation to prevent androgen-stimulated proliferation and an eventual progression to a more malignant and metastatic form of prostate cancer.

Early detection of prostate cancer has more recently become possible with the development of sensitive tests for circulating antigens that are diagnostic for prostate cancer. Such antigens include prostate serum antigen (PSA), prostatic acid phosphatase (PAP), and prostatic inhibin (PIP). Early detection affords the opportunity for early treatment. Currently, the most common available methods of treating prostate cancer include surgery or radiotherapy to eliminate the cancerous prostate gland. In addition, various methods of hormone therapy (*e*.*g*., androgen ablation or control) are commonly employed to slow the development of solid and metastatic prostate cancer. However, patients subjected to these currently available therapies for treating prostate cancer are also at risk for various undesirable side effects, notably, incontinence and impotence. Thus, there is a need for a therapy that is not associated with such undesirable side effects.

Schneyer and co-workers previously studied the effect of the protein activin, a non-morphogen member of the TGF-β superfamily, as a therapeutic agent to treat prostate cancer. See, *e*.*g*., Wang et al., Endocrinology 137: 5476-5483 (1996). However, high renal toxicity has thwarted further development of activin as an effective therapeutic anti-cancer agent. As described herein, it has now been found that morphogens, such as OP-1 and BMP-6, are capable of inhibiting proliferation of both androgen-dependent and androgen-independent prostate cancer cells. In contrast to activin, morphogens such as OP-1 and BMP-6 are known to be compatible with bone tissue, kidney function, and other soft tissues of the body. Accordingly, this invention provides a method of treating prostate cancer comprising administering a morphogen, such as OP-1 or BMP-6, to a prostate cancer patient. Once a morphogen has been administered to a patient, morphogen-mediated inhibition of prostate cancer cell proliferation may be monitored by a clinician using various standard methods, including examination to detect a decrease in tissue size and texture, measuring of the levels of circulating antigens, such as PSA, and monitoring of patient discomfort and genito-urinary tract function.

As shown herein, morphogens, such as BMP-6 and OP-1, inhibit proliferation of prostate cancer cells as demonstrated using the androgen-responsive LNCaP cell line, an established *in vitro* model of androgen-responsive prostate cancer. The LNCaP prostate cancer model was employed in three experiments. First, the effect of BMP-6, OP-1 (BMP-7), Activin, or TGF-β1 on cell growth of LNCaP cells in the absence of exogenous androgen was examined. Second, the effect of BMP-6, OP-1 (BMP-7), Activin, or TGF-β1 on cell growth of LNCaP cells exposed to androgen was examined. Third, the effect of follistatin, an activin and morphogen binding protein, on the bioactivity of BMP-6, OP-1 (BMP-7), and Activin in LNCaP cells was examined.

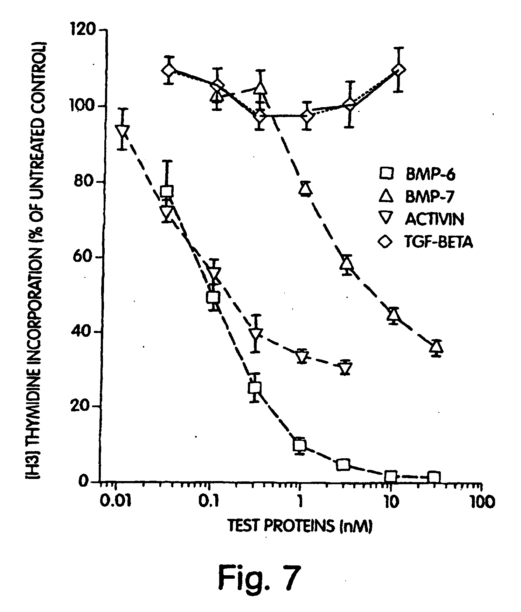

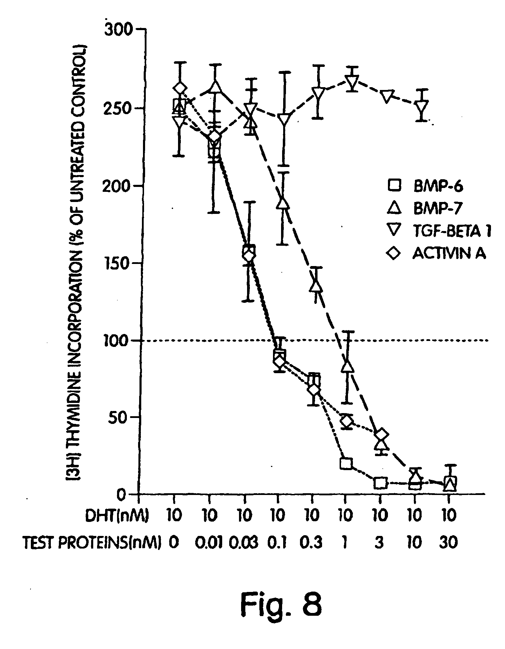

LNCaP cells were obtained from the American Type Culture Collection (Rockville, MD) and grown in 75 cm flasks with RPMI-1640 containing 10% fetal bovine serum (RPMI/10%FBS) in a humidified atmosphere of 5% CO₂:95% air. Culture medium was changed every third day. For assay of activin and BMPs the stock cultures were subcultured by replating trypsinized cell suspensions onto 96 well plates. For this purpose, LNCaP cells were plated in 24 well plates at 3x10⁴ cells/well in 0.5 mL of RPMI/10% FBS, and incubated for 48 hours to establish subconfluent cultures for study. After the preincubation period, the medium was gently aspirated, the cells were washed once with warm RPML, and the cells were subsequently cultured in 0.5 mL of RPMI containing 2% FBS for the study of treatment effects on cell proliferation rate (*e*.*g*., cell growth). Varying doses of activin, BMPs, DHT, or FS, alone or in combination, were added to triplicate wells and incubations were continued for another 48 hours. After [³H]-thymidine incorporation into cells, the cells were washed once with RPMI and precipitated with 0.5 mL of 10% cold trichloroacetic acid (TCA) for 20 minutes at 4°C. After two methanol washes, the precipitate was solubilized in 0.5 mL of 1 N sodium hydroxide and the amount of radioactivity was counted in a liquid scintillation counter. [³H]-thymidine incorporation is expressed as cpm/well and represents a quantitative measurement of cell proliferation rate. In the first experimental paradigm, LNCaP cells (in the absence of exogenous androgen) were exposed to various concentrations of BMP-6 OP-1 (BMP-7), Activin, or TGF-β1 as indicated. The effect of these substances on LNCaP cells was assessed using a [³H]-thymidine uptake assay. FIG. 7 shows that activin and the morphogens, BMP-6 and OP-1 (BMP-7), inhibit cell growth in a dose dependent manner. BMP-6 had a higher potency than OP-1 (BMP7). In the second experimental paradigm, LNCaP cells (exposed to 10 nM DHT androgen) were exposed to various concentrations of BMP-6, OP-1 (BMP-7), Activin or TGF-β1 in the presence of androgen (10 nM DHT) and the effect of these substances, was assessed using [³H]-thymidine uptake assay. FIG. 8 shows that activin and the morphogens (BMP-6 and OP-1 (BMP-7), inhibited proliferation of the androgen-stimulated LNCaP cells in a dose dependent manner. BMP-6 had a higher potency than OP-1 (BMP-7).

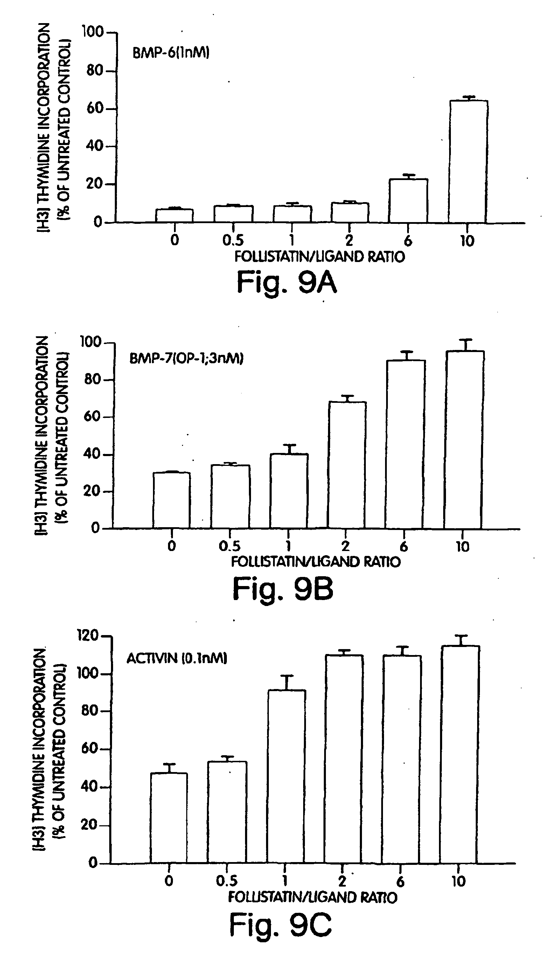

In the third experimental paradigm, LNCaP cells were exposed to 1 nM BMP-6, 3 nM OP-1 (BMP-7), or 0.1 nM Activin and subsequently were exposed to the activin/BMP-binding protein follistatin (FS) in a ratio of follistatin: ligand of 0.5:1, 1:1, 2:1, 5:1, and 10:1. The effects of follistatin and ligand on proliferation of the LNCaP cells were assessed using [³H]-thymidine uptake assay. FIG. 9 shows that follistatin neutralized the bioactivity of both morphogens, BMP-6 and OP-1 (BMP-7).

### Example 2 Morphogen Inhibition of Proliferation and Induction of Differentiation of 5 Estrogen Responsive Human Breast Cancer MCF-7 Cells

This example demonstrates that the morphogen, OP-1, inhibits growth of estrogen responsive human breast cancer MCF-7 cells. MCF-7 cells were grown in monolayer cultures in Eagle's MEM supplemented with 5% fetal bovine serum (FBS), 2 mM L-glutamine, Earle's BSS, 1 mM sodium pyruvate, antibiotics, and 10 mg/ml bovine insulin. To prevent possible steroid-like artifacts from phenol red containing medium, before experimental treatments, the cells were incubated with phenol-free, serum-free medium or phenol-free medium supplemented with 5% FBS, each stripped of endogenous steroids with dextran-coated charcoal.

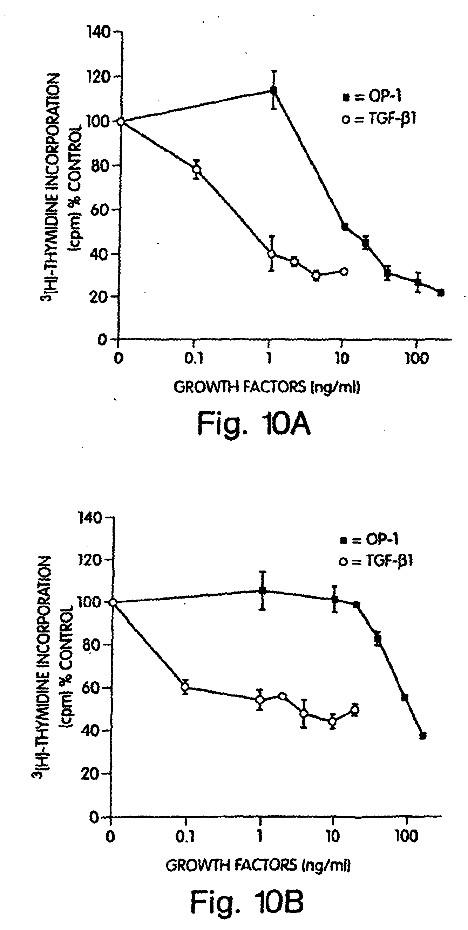

In the first experiment, the effect of OP-1 and TGF-β on proliferation of MCF-7 cancer cells was tested in the presence and absence of serum. The cells were grown in 24- or 48 well plates. OP-1 or TGF-β1 was added at various concentrations, as indicated, in serum free medium, or, alternatively, OP-1 or TGF-β1 was added in 5% serum containing medium at various concentrations, as indicated. FIGS. 10A and 10B show that both OP-1 and TGF-β1 inhibited cell growth in a dose dependent manner using a [³H]-thymidine uptake assay. In the presence or absence of serum, OP-1 inhibited cell growth by approximately 80% of control at 200 ng/ml (FIG. 10A, filled squares) and by approximately 65% of the control at 200 ng/ml in the presence of 5% serum containing medium (FIG. 10B, filled squares). TGF-β1 inhibited cell growth by approximately 65% of control at 10 ng/ml in serum free medium (FIG. 10A,unfilled circles) and by approximately 55% of control at 10 ng/ml in 5% serum containing medium (FIG. 10B, unfilled circles).

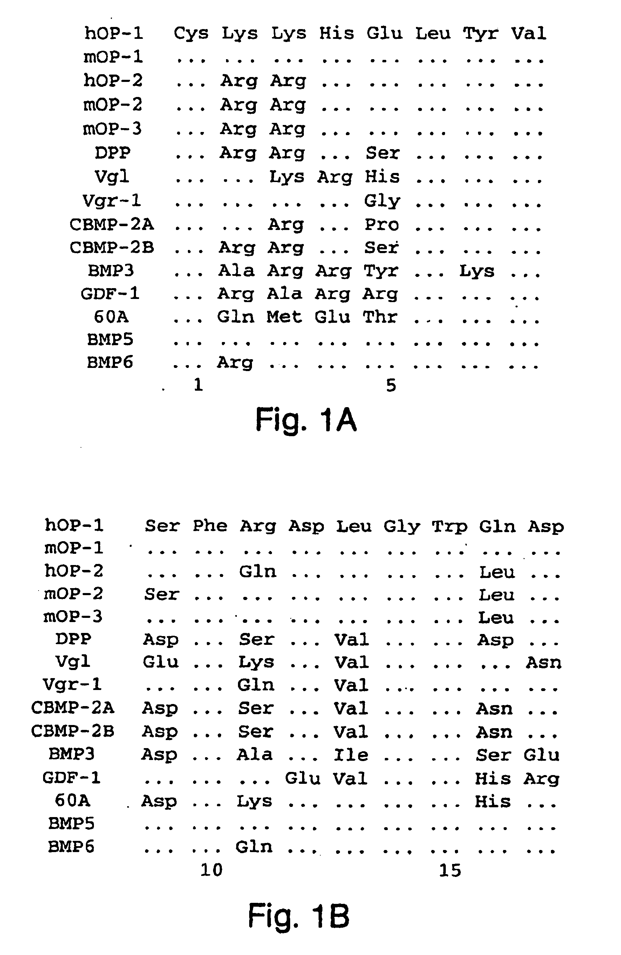

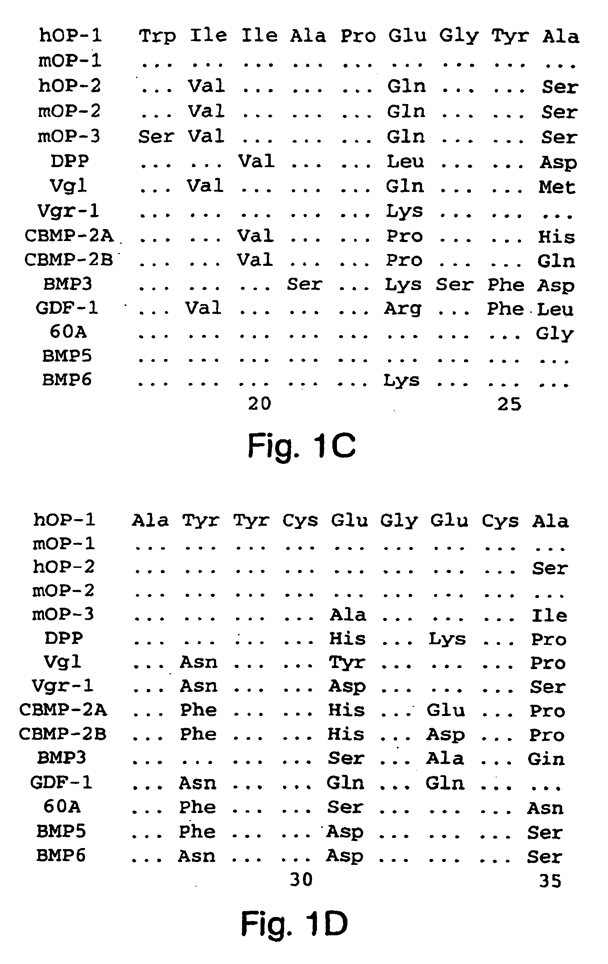

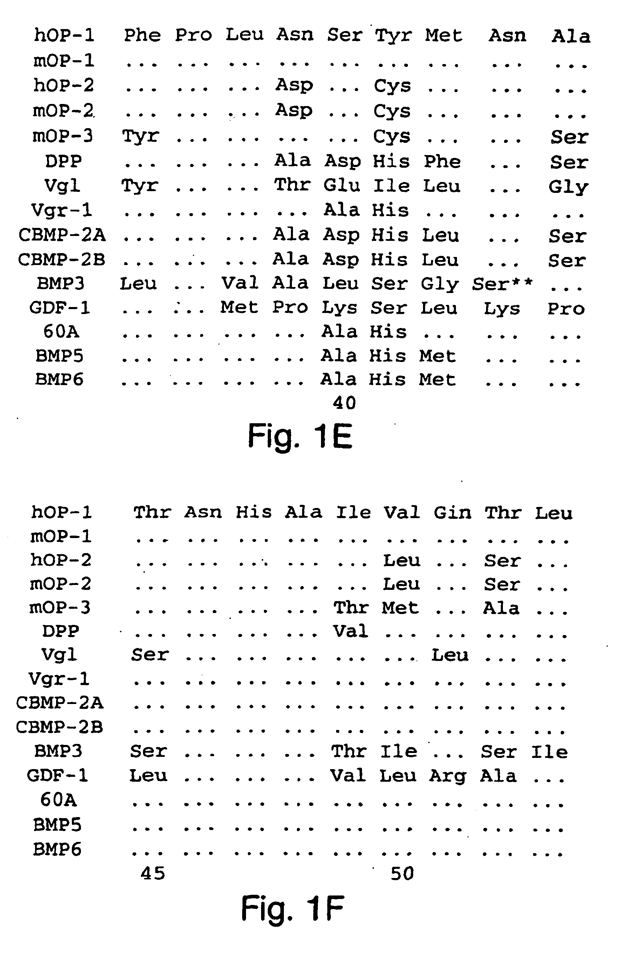

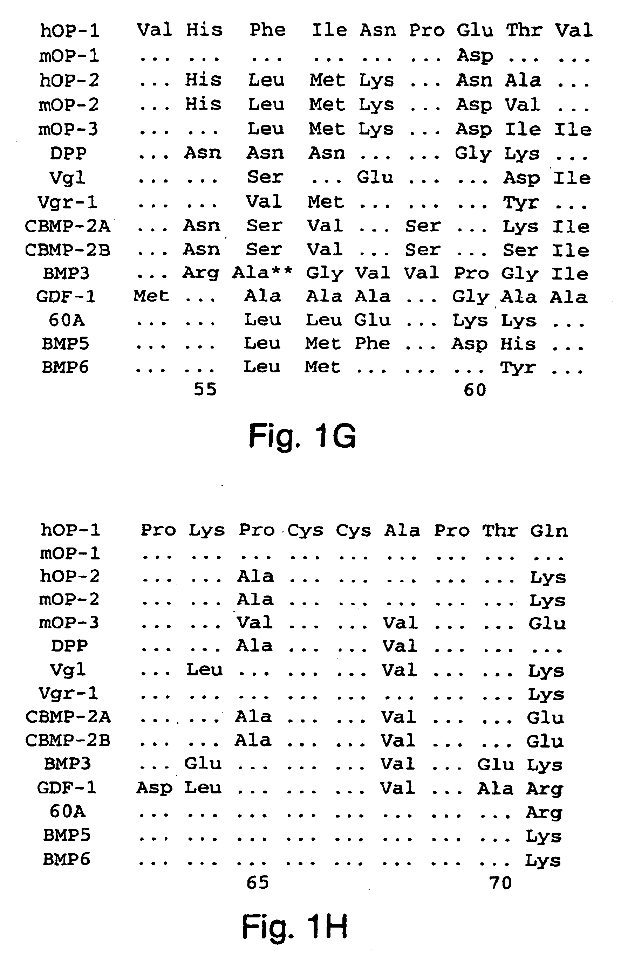

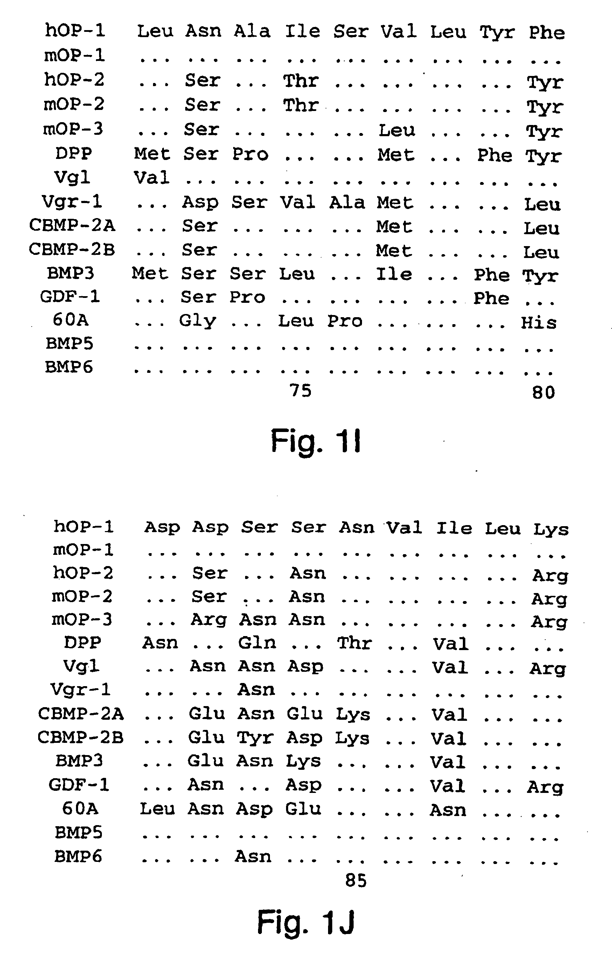

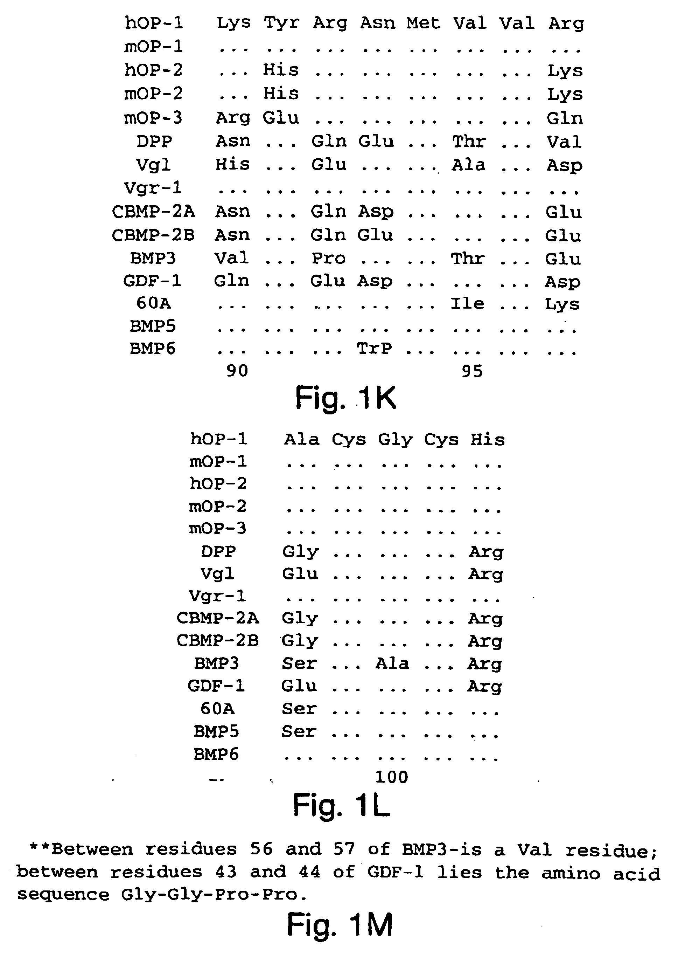

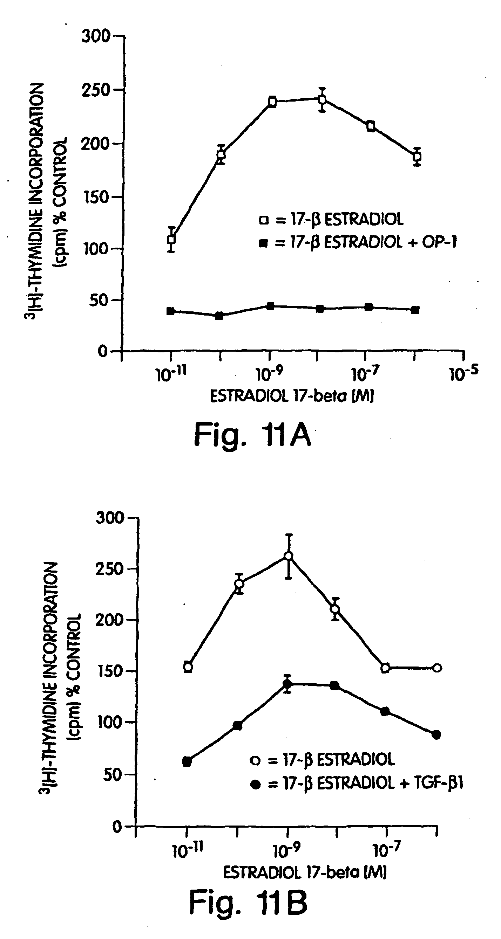

In a related experiment, the effects of OP-1 or TGF-β1 on 17-β estradiol-stimulated MCF-7 breast cancer cells were examined. The MCF-7 cells were incubated in medium supplemented with 17-β estradiol (1 x 10⁻¹¹, 10⁻¹⁰, 10⁻⁹, 10⁻⁸, 10⁻⁷, 10⁻⁶ M) and then exposed to 200 ng/ml of OP-1. The effects on cell growth were assayed using a [³H]-thymidine uptake assay. FIG. 1A shows that while MCF-7 cells exposed to 17-β estradiol and not OP-1 had an increase of cell growth of approximately 250% of control at 10⁻⁹ M 17-β estradiol (unfilled squares). Those MCF-7 cells exposed to both 10⁻⁹ M 17-β estradiol and OP-1 showed an inhibition of cell growth of approximately 80% of control (filled squares). Using the same protocol described immediately above, 10 ng/ml TGF-β1 was substituted for OP-1. FIG. 11B shows that TGF-β inhibited cell growth by approximately 40% of control in MCF-7 cells stimulated with 10⁻¹¹ M 17-β estradiol (filled circles).

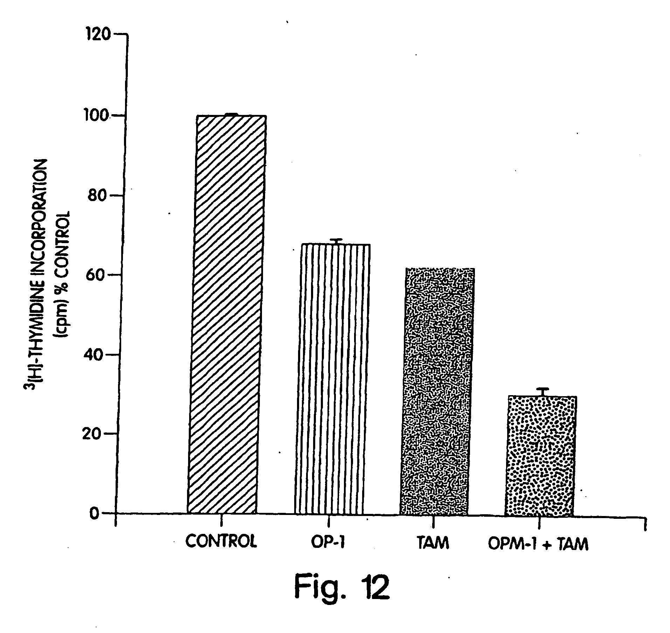

In another experiment, tamoxifen, an estrogen antagonist that is known to inhibit growth of MCF-7 cells, was examined for its effects in comparison to as well as in addition with OP-1. MCF-7 cells were exposed to 1 x 10⁻⁶ M tamoxifen, 100 ng/ml of OP-1, or both tamoxifen and OP-1 combined. Cell growth was assayed using a [³H]-thymidine uptake assay. FIG. 12 shows that OP-1 or tamoxifen at these concentrations inhibited cell growth by approximately 40% of control. Together, OP-1 and tamoxifen inhibited cell growth by approximately 70% of control.

In another related experiment, the OP-1 gene was stably transfected into MCF-7 cells using standard calcium phosphate precipitation. The major immediate early promoter (MIE) of Cytomegalovirus, isolated from plasmid pCDM8 (Invitrogen), was used to direct the transcription of the OP-1 cDNA. Both SV4O early region splice signals and poly-A addition signals obtained from pMAMneo (Clonetech, Inc.) were added as 3' processing elements. A shorter 3' region derived from the same vector, without the intron and splice sites, also has been used with equal success. The eukaryotic selection marker neomycin also was present, under the transcriptional control of SV40 early promoter. See U.S. Patent No. 5,712,119; U.S. Patent No. 5,614,385; U.S. Patent No. 5,585,237.

One day prior to transfection, cells were plated at a density of 1 x 10⁶ cells/100 mm petri dish. Fifty micrograms of purified DNA prepared from the plasmid containing the OP-1 gene was transfected into the cells by the calcium phosphate precipitation method. Two days post-transfection, cells were trypsinized and plated 1:3 onto fresh plates containing selection medium containing 400 mg/ml G418. G418 resistant colonies were visible about two weeks later, and some of these cells were used to verify OP-1 expression by Northern Blot analysis.

When MCF-7 cells were transfected with the OP-1 gene, a change in morphology(fibroblast-like) occurred. Cellular responsiveness to estrogen also was altered. The OP-1 transfected MCF-7 cells were treated with various doses of 17-β estradiol for 3 days (10⁻⁷-10⁻¹¹ M 17-β estradiol). Cell growth was assayed using a [³H]-thymidine uptake assay. 17-β estradiol failed to stimulate cell growth. In fact, MCF-7 cells transfected with the OP-1 gene showed a dose-dependent inhibition of cell growth of approximately 50% of control at 1x10⁻⁷ M 17-β estradiol.

These findings collectively demonstrate that OP-1 inhibits both basal and estrogen stimulated growth of MCF-7 breast cancer cells. OP-1 also induces cellular differentiation in MCF-7 cells, possibly a switch from epithelial cell type to mesenchyme, consistent with the use of morphogens such as OP-1 as a therapeutic agent for certain estrogen-responsive human breast cancers.

### Example 3 Morphogen-induced Inhibition of Tumor Cell Proliferation in vitro

The effect of morphogen on the proliferation of two murine prostate cancer cell lines was examined *in vitro.* Initially, the expression of BMP receptors (BMPRs) in these mouse prostate cancer cell lines was investigated by Northern blot analysis. The results demonstrated that both cell lines express three types of BMPRs and that a morphogen, BMP6, inhibits the proliferation of these cell lines in a dose-dependent manner.

### Cell Culture

Two cell lines, derived from prostate cancer of TRAMP (Transgenic Adenocarcinoma of Mouse Prostate) mice and designated E4 and F11, were maintained in Dulbecco's Modified Eagle Medium (DMEM) supplemented with 10% Fetal Bovine Serum (FBS) 5 µg/ml inculin, and 10⁸ M dihydroresterone (*see* Foster et al., Cancer Res. 7: 3325-330 (1997)). All cells were derived from the 20^{th} through 23^{rd} passages.

Prior to treatment with BMP-6, the media were switched to DMEM supplemented with 1% FBS. Then BMP-6 was added at following concentrations: 1, 10, and 100 µg/ml. The media were changed every other day. At the end of a 6-day period, cells were harvested and counted using a hemacytometer. All assays were performed three times and similar results were obtained each time.

### Messenger RNA Isolation and Northern Blot Analysis

The expression of BMPRs in E4 and F11 cells was investigated by Northern blot analysis. After harvesting the cells, mRNA was isolated using a commercial kit (Ribosep, Collaborative Biomolecules, Bedford, MA) according to the manufacturer's recommended protocol. For Northern blot analysis, 2 pg of poly A RNA were separated by electrophoresis in 1% formaldehyde-agarose gel and transferred to nylon membranes (Zeta-Probe GT membrane, Bio-Rad Laboratories, Hercules, CA). The membranes were subsequently rinsed in 2X SSC (1X SSC = 0.15 M Na Citrate, pH 7.0). Then the separated RNA was cross-linked to the nylon membrane using an ultraviolet light cross linker. Prehybridization was performed in 50% formamide, 0.12 M Na₂HPO₄ (pH 7.2), 0.25 M NaCl, 7% (wt/vol) SDS, and 250 µg/ml heat-denatured herring sperm DNA (Promega Corp. Madison, WI) for 2 hrs at 42°C. Hybridization was performed overnight at 42°C in then prehybridization solution containing probe labeled with [³²P]-deoxy-CTP (Amersham Corp., Arlington Heights, IL) using a random oligonucleotide priming kit (Random Primers DNA labeling system, Life Technologies). After washing the membranes in sequential dilutions of SSC, autoradiography was carried out using Kodak X-Moat AR film (Eastman Kodak, Rochester, NY) with an intensifying screen at -70°C.

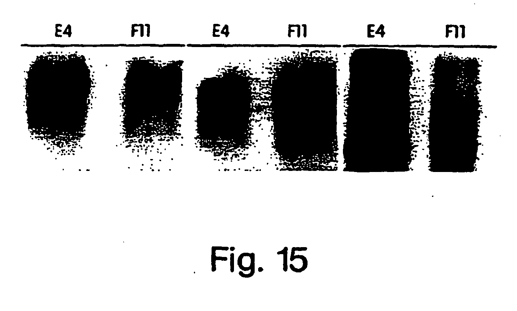

As shown in FIG. 15, both cell lines expressed the three types ofBMPRs: BMPR-IA, BMPR-IB, and BMPR-II. As previously reported, there were three splice variants for BMPR-II. *See* Rosenzweig et al., Proc. Natl. Acad. Sci. USA 92: 7632-636 (1995).

### Inhibition of Proliferation

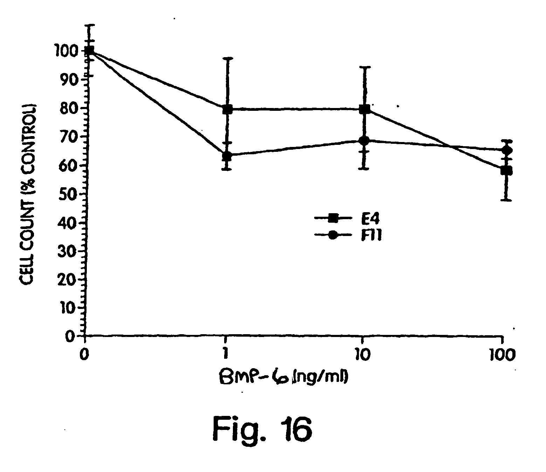

The addition of BMP-6 to these cell lines in tissue culture dramatically decreased the rate of proliferation in a dose-dependent manner (see FIG. 16). Specifically, in the presence of 100 ng/ml BMP-6, the cell count of both E4 and F11 cells were approximately 60% of that of the control (no BMP-6). Since the two cell lines expressed all three types ofBMPRs and were sensitive to BMP-6, E4 cell line was chosen randomly for subsequent analysis concerning the therapeutic potential of BMP-6, *in vivo*.

### Example 4 Morphogen-induced Inhibition of Tumor Progression in vivo

The ability of morphogen to inhibit the progression of cancer *in vivo* was tested using the murine prostate cancer cell line E4, as discussed in Example 7. Adult male C57BL/6 mice were purchased and maintained according to the NH standards established in the Guidelines for the Care and Use of Experimental Animals. Thirty mice were inoculated with 10⁷ cells subcutaneously. At 6 weeks, 21 mice had palpable tumor. These animals were divided into three groups of 7 each. The experimental group received 200 µl of 100 ng/ml BMP-6 while the control groups received either the vehicle (20 mM acetate and 5% Mannitol, pH 4.5) or no treatment. Injections were delivered directly to the tumors once every other day for 3 weeks. The animals were observed for 10 weeks after the initiation of therapy, and tumor volume was measured in each animal weekly.

At the end of the ten-week period, all tumors were harvested and preserved in formalin and embedded in paraffin. Subsequently, 5 µm sections were obtained from each tumor and stained with hematoxylin and eosin (H&E).

### Inhibition of tumor progression

At the end of the 10-week period after the initiation of treatment, 5 of 7 mice treated with BMP-6 were alive while all animals in the other group were dead (Table 1). Of the five animals, two had unpalpable tumor.

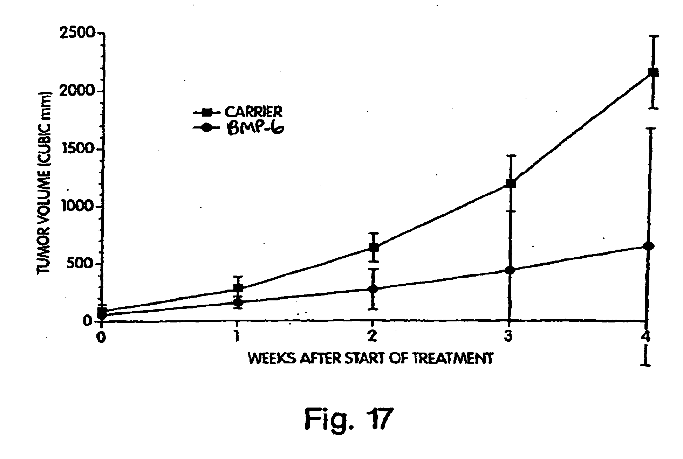

In addition to the rate of survival, BMP-6 treatment also decreased the rate of tumor growth. The results, shown in FIG. 17, demonstrate that the average tumor in mice treated with BMP-6 was approximately one third of that in mice given vehicle only. There was no significant difference between the rate of tumor growth between the group that received the vehicle and the animals that had no treatment (data not shown).

**Table 1.**

| Rate of Survival of Mice Treated with BMP-6 | | |
|---|---|---|
| **Innoculum** | **Survival No./Total No. %** | **P-value** |
| None | 0/7(0) | |
| Vehicle | 0/7 (0) | |
| BMP-6 | 5/7 (71)** | <0.001 |

| | | |
|---|---|---|
| *Survival at 10 weeks after tumor became palpable. **Two had unpalpable tumor while 2 had necrotic center. | | |

### Data Analysis

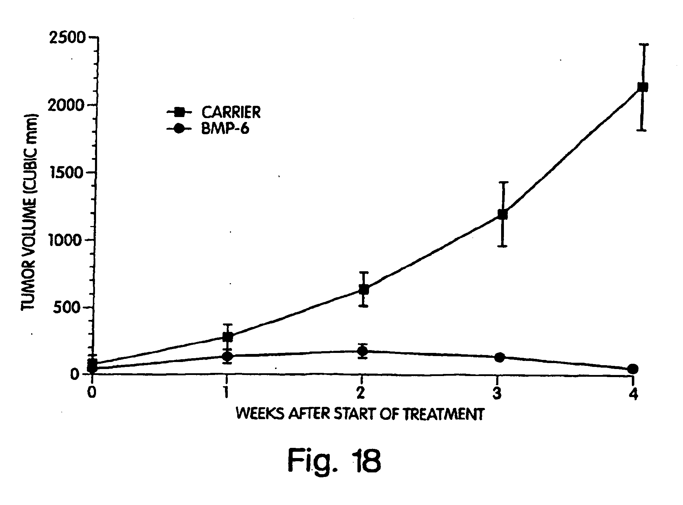

As an attempt to determine the effect of morphogen treatment on mice that responded to the treatment, re-analysis of the data was carried out after removing those from the two non-surviving animals, which did not respond to BMP-6 treatment (FIG. 18). All statistics were evaluated by the χ² test, and P<0.05 was considered statistically significant. The new data analysis indicates that tumor volume actually started to decrease after 2 weeks of BMP-6 injection in animals that responded to the treatment. At the end of 4 weeks after the initiation of BMP-6 administration, the average tumor size of the mice that responded was approximately 5% of that of the control group that received the vehicle only.

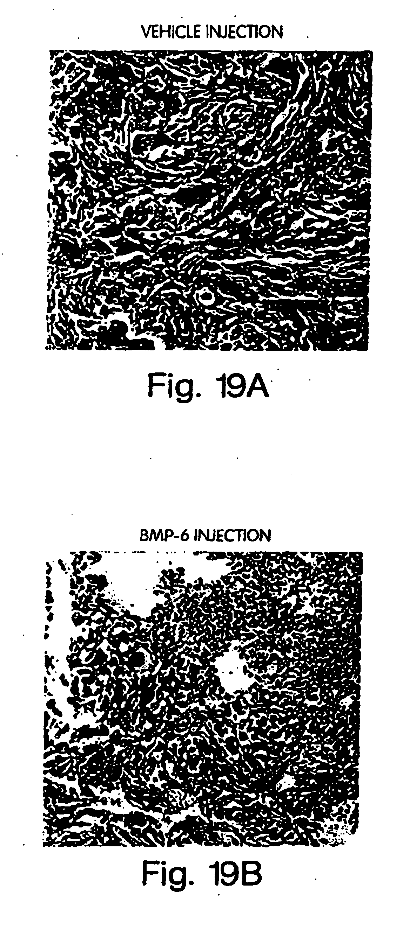

As an initial attempt to determine the mechanism underlying the morphogen-induced tumor regression seen in the present study, the tumors were harvested and stained with H&E for microscopy. As shown in FIGS. 19A and 19B, tumors that were treated with BMP-6 showed severe necrosis and diffuse inflammation.

### IV. COMPARATIVE EXAMPLES FALLING OUTSIDE THE SCOPE CLAIMED

### Comparative Example 1 Morphogen-Induced Cell Differentiation of NG108-15 Neuroblastoma/Glioma Cell Line

As one example of morphogen-induced cell differentiation, the effect of OP-1 on the differentiation of neuronal cells was tested in culture. Specifically, the effect of OP-1 on the NG108-15 neuroblastoma x glioma hybrid clonal cell line was assessed. The NG108-15 cell line (also referred to as simply "NG108") displays a fibroblastic-type morphology in culture. The cell line can be induced to differentiate chemically using 0.5 mM butyrate, 1% DM50 or 500 mM Forskolin, inducing the expression of virtually all important neuronal properties of cultured primary neurons. However, chemical induction of these cells also induces cessation of cell division.

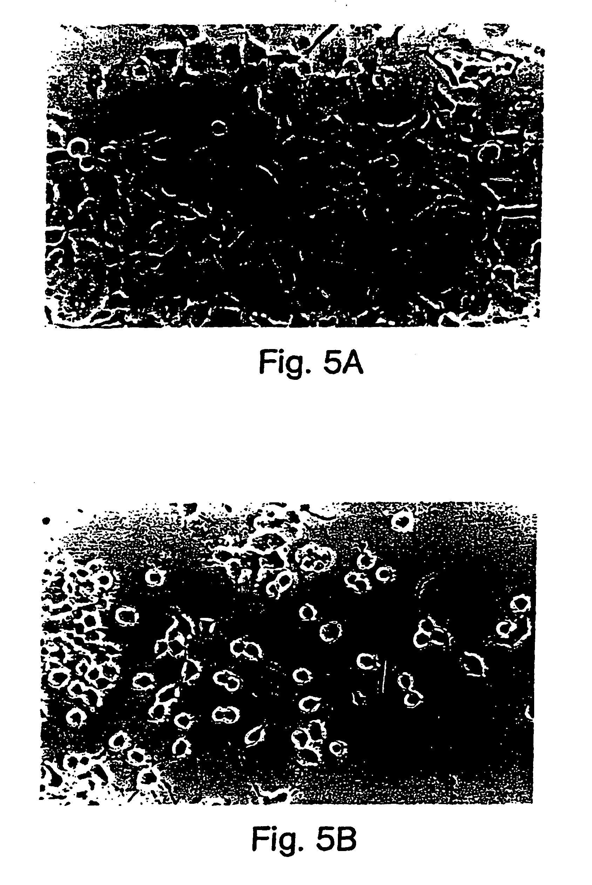

In the present experiment NG108-15 cells were subcultured on poly-L-lysine coated 6-well plates. Each well contained 40,000-50,000 cells in 2.5 ml of chemically defined medium. This chemically defined medium contained Dulbecco's modified Eagle's medium: Ham's F-12 (3:1), NaHCO₃ (3.7 g/liter), trace elements (0.5 nM Mo, 0.5 nM Mn, 5 nM Cd, 15 nM Se, 25 nM Sn, 0.25 nM Ni, and 0.25 nM Si), 25 mM HEPES, 10 :g oleic acid complexed to fatty acid-free albumin, 50 µg/ml transferrin, 25 µg/ml insulin, 2 mM glutamine, and 25 nM Na₃VO₄. See Chamess et al., J. Biol. Chem. 261: 3164-3169 (1986); Perides et al., Proc. Natl. Acad. Sci. USA 89: 10326-30 (1992). On the third day, 2.5 :1 of OP-1 in 60% ethanol containing 0.025% trifluoroacetic was added to each well. OP-1 concentrations of 0, 1, 10, 40 and 100 ng/ml were tested. The media was changed daily with new aliquots of OP-1. After four days with 40 and 100 ng OP-1/ml concentrations, OP-1 induced differentiation of NG108 cells. FIG. 5 shows the morphological changes that occur. The OP-1 induced clumping and rounding of the cells and the production of neurite outgrowths characteristic of the neuronal phenotype (compare FIG. 5A (naive NG108 cells) with FIG. 5B, showing the effects of OP-1 treated cells). Thus, the OP-1 can induce the cells to differentiate into a neuronal cell morphology. Some of the outgrowths appeared to join in a synaptic-type junction. This effect was not seen in cells incubated with TGF-β1 at concentrations of 1 to 100 ng/ml. The neuroprotective effects of OP-1 were demonstrated by comparison with chemical differentiation agents on the NG108 cells. 50,000 cells were plated on 6-well plates and treated with butyrate, DMSO, Forskolin or OP-1 for four days. Cell counts demonstrated that in the cultures containing the chemical agents the differentiation was accompanied by a cessation of cell division. In contrast, the cells induced to differentiate by OP-1 continued to divide, as determined by [³H]-thymidine uptake. The data suggest that OP-1 is capable of maintaining the stability of the cells in culture after differentiation.

### Comparative Example 2 Morphogen-Induced Differentiation and Inhibition of Proliferation of a Human Teratocarcinoma Cell Line

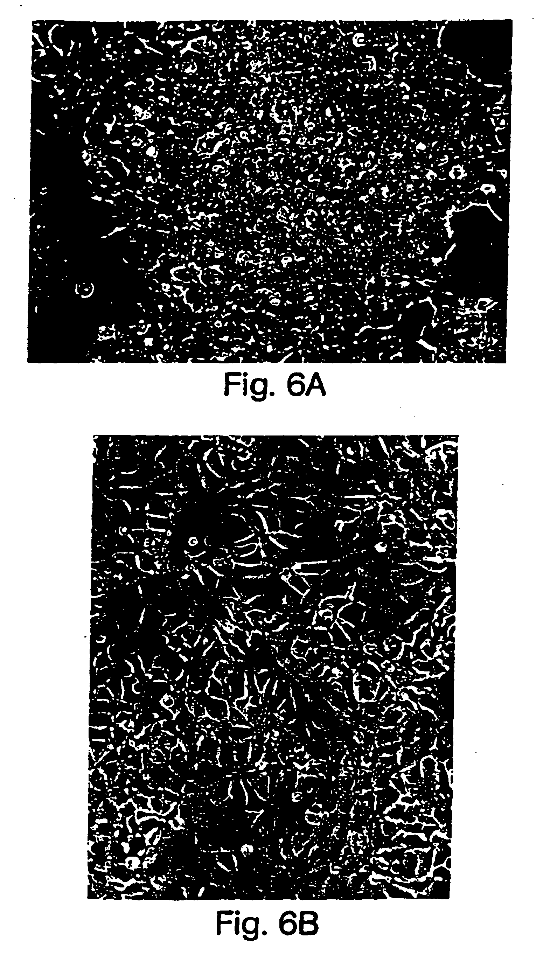

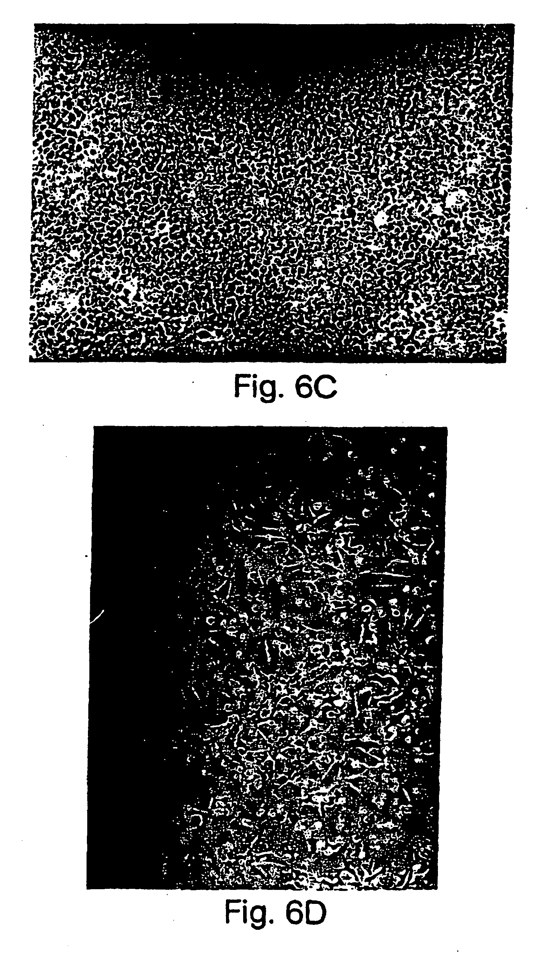

Another related example demonstrates the ability of the morphogens of the invention to induce the "redifferentiation" of transformed cells. The human embryonal carcinoma (EC) cell line NTERA-2CL.D1 ("NTERA2") is derived from a human teratocarcinoma. *See* Andrews et al. Lab. Invest. 50: 147-167 (1984). In the absence of an external stimulant these cells can be maintained as undifferentiated stem cells, and can be induced to grow in serum free media(SFM). In the absence of morphogen treatment, the cells proliferate rampantly and are anchorage-independent. The effect ofmorphogen treatment is seen in FIGS. 6A-D. FIGS. 6A and 6B show 4 days of growth in SFM in the presence of OP-1 (25 ng/ml, 6A) or the absence of morphogen (6B). FIGS. 6C and 6D are 5 days growth in the presence of 10 ng/ml OP-1 (6C) or no morphogen (6D). FIGS. 6C and 6D are at 10x and 20x magnification compared to FIGS. 6A and 6B. As can readily be seen, in the presence of OP-1, the EC cells grew as flattened cells, becoming anchorage dependent. In addition, growth rate was reduced approximately 10-fold. Finally, the cells were induced to differentiate. These results indicate that in a teratocarcinoma cell line, morphogens differentiate and inhibit growth of the cells.

### Comparative Example 3 Morphogen Inhibition of Proliferation of Human Glioblastoma A-172 Cells

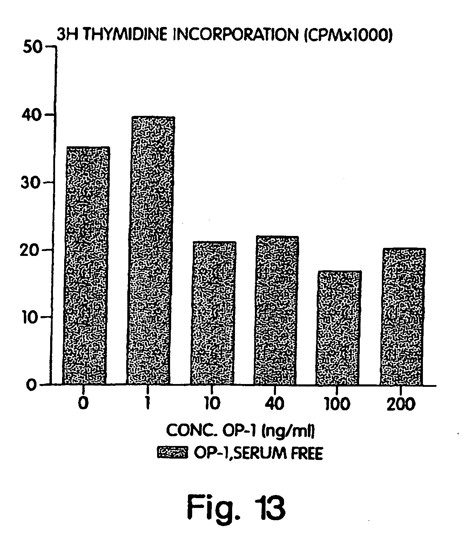

This example demonstrates that the morphogen, OP-1, inhibits cell growth of human glioblastoma A-172 cells. A-172 cells were plated 5 x 10⁴ cells per well in a 48-well plate in DMEM containing 10% FBS. After 24 hours, the growth medium was replaced with serum free DMEM. OP-1 (1, 10, 40, 100, 200 ng/ml) was added to the cells and incubated at 37°C in the presence of 5% CO₂ for 24 hours, which included a 6 hour, pulse with 1 µCi [³H]-thymidine. After the incubation, the cells were washed with PBS. TCA (10%) was added and the cells were incubated for 15 minutes. The cell layer was washed three times with distilled water and was extracted with 1% SDS. The incorporated radioactivity was measured in a liquid scintillation analyzer. FIG. 13 shows that the morphogen, OP-1, inhibited the proliferation of A-172 cancer cells by approximately 50% of control at 100 ng/ml.

### Comparative Example 4 Morphogen Inhibition of Proliferation of Human Lung Carcinoma A-549 Cells

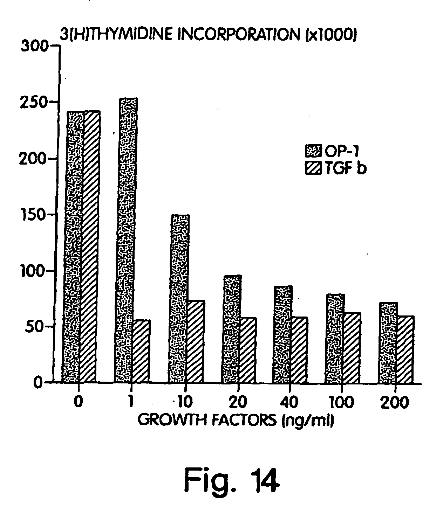

This example demonstrates that the morphogen, OP-1, inhibits cell growth of human lung carcinoma A-549 cells. A-549 cells were plated 5 x 10⁴ cells per well in a 48-well plate in DMEM containing 10% FBS. After 24 hours, the growth medium was replaced with serum free DMEM. OP-1 (1, 10, 20, 40, 100, 200 ng/ml) was added to the cells and incubated at 37°C in the presence of 5% CO₂ for 24 hours, which included a 6 hour pulse with 1 µCi [³H]-thymidine. After the incubation, the cells were washed with PBS. 10% TCA was added and the cells were incubated for 15 minutes. The cell layer was washed three times with distilled water and was extracted with 1% SDS. The incorporated radioactivity was measured in a liquid scintillation analyzer. FIG. 14 shows that OP-1 inhibited the cell growth of A-549 1mg cancer cells in a dose dependent manner with a 70% inhibition achieved at 200 /ml. TGF-132, on the other hand, maximally inhibited 77% of cell growth at 1 ng/ml.

## Claims

1. Use of a morphogen comprising a dimeric protein, wherein the morphogen is selected from OP-1 and BMP-6, in the manufacture of a medicament for alleviating the symptoms of prostate cancer, and wherein the morphogen inhibits growth of cancer cells.

2. Use of a morphogen comprising a dimeric protein, wherein the morphogen is selected from OP1 in the manufacture of a medicament for alleviating the symtoms of breast cancer, and wherein the morphogen inhibits growth of cancer cells.

3. Use of claim 1, wherein the morphogen is OP-1.

4. Use of claim 1, wherein the morphogen is BMP-6.

5. Use of any one of the preceding claims, wherein the morphogen is present in a pharmaceutically acceptable vehicle.

6. Use of claim 5, wherein the vehicle is selected from the group consisting of biocompatible microspheres, biodegradable microspheres, and bioerodable microspheres.

7. Use of claim 5, wherein the vehicle is selected from the group consisting of biocompatible matrices, biodegradable matrices, and bioerodable matrices.

8. Use of claim 5, wherein the vehicle is an aqueous solution.

9. Use of claim 1 or 2, wherein the medicament comprises cells capable of expressing the morphogen.

10. Use of claim 1 or 2, wherein the medicament comprises a non-infectious, nonintegrating DNA encoding the morphogen operably linked to a promoter, wherein the DNA is free from association with transfection-facilitating proteins, viral particles, liposomal formulations, charged lipids, and calcium phosphate precipitating agents.

11. Use of claim 10, wherein the non-infectious, non-integrating DNA encoding the morphogen operably linked to promoter further comprises an enhance element in operative association with the promoter.

12. Use of claim 1 or 2, wherein the symptoms are selected from the group consisting of abnormal bodily function, abnormal cell morphology, abnormal enzyme levels, abnormal hormone levels, abnormal oncofetal antigen levels, abnormal tissue growth, abnormal tissue mass, abnormal tumour-associated protein levels, altered neurologic function, altered neurologic structure, angiogenesis, bleeding, cells having a cancer cell phenotype, diarrhea, effusions, fatigue, fever, lesions, malnutrition, metastasis, nausea, obstruction of a bodily passageway, opportunistic infection, pain, poor Kamofsky performance status, presence of cell-surface markers, presence of histological markers; presence of intracellular markers, presence of molecular markers, tumour invasion, unregulated cell proliferation, urinary frequency, and weight loss.

13. Use of claim 1 or 2, wherein the morphogen inhibits cellular incorporation of [³H]-thymidine in an in vitro assay.

14. Use of claim 1 or 2, wherein the medicament further comprises at least one substance in combination with the morphogen.

15. Use of claim 14, wherein the substance is an anti-cancer agent.

16. Use of claim 1 or 2, wherein the cancer cells express at least one bone morphogenic protein receptor (BMPR) specific for the morphogen.

17. Use of claim 16, wherein the BMPR is selected from the group consisting of BMPR-IA, BMPR-IB, BMPR-II, and splice variants thereof.

18. Use of claim 1 or 2, wherein the morphogen reduces tumour size or development.

19. Use of claim 1 or 2, wherein the morphogen increases survival rate.

20. Use of claim 1 or 2, wherein the morphogen results in tumour necrosis and diffuse inflammation.

## Patentansprüche

1. Verwendung eines Morphogens mit einem dimeren Protein, wobei das Morphogen aus OP-1 und BMP-6 ausgewählt ist, bei der Herstellung eines Medikaments zur Linderung der Symptome von Prostatakrebs, wobei das Morphogen das Wachstum von Krebszellen inhibiert.

2. Verwendung eines Morphogens mit einem dimeren Protein, wobei das Morphogen aus OP-1 ausgewählt ist, bei der Herstellung eines Medikaments zur Linderung der Symptome von Brustkrebs, wobei das Morphogen das Wachstum von Krebszellen inhibiert.

3. Verwendung nach Anspruch 1, wobei das Morphogen OP-1 umfasst.

4. Verwendung nach Anspruch 1, wobei das Morphogen BMP-6 umfasst.

5. Verwendung nach einem der vorhergehenden Ansprüche, wobei das Morphogen in einem pharmazeutisch akzeptablen Vehikel vorliegt.

6. Verwendung nach Anspruch 5, wobei das Vehikel aus der Gruppe ausgewählt ist, die umfasst biologisch kompatible Mikrosphären, biologisch abbaubare Mikrosphären und biologisch erodierbare Mikrosphären.

7. Verwendung nach Anspruch 5, wobei das Vehikel aus der Gruppe ausgewählt ist, die umfasst biologisch kompatible Matrizen, biologisch abbaubare Matrizen und biologisch erodierbare Matrizen.

8. Verwendung nach Anspruch 5, wobei das Vehikel eine wässrige Lösung ist.

9. Verwendung nach Anspruch 1 oder 2, wobei das Medikament Zellen umfasst, die in der Lage sind, das Morphogen zu exprimieren.

10. Verwendung nach Anspruch 1 oder 2, wobei das Medikament eine nicht infektiöse, nicht integrierende DNA umfasst, die das Morphogen, das funktionsfähig mit einem Promotor verknüpft ist, kodiert, wobei die DNA frei von einer Verbindung mit Transfektionen erleichternden Proteinen, viralen Partikeln, liposomalen Formulierungen, geladenen Lipiden und Calciumphosphat abscheidenden Wirkstoffen ist.

11. Verwendung nach Anspruch 10, wobei die nicht infektiöse, nicht integrierende DNA, die das Morphogen, das funktionsfähig mit einem Promotor verknüpft ist, kodiert, ferner ein Enhancer-Element in funktionsfähiger Verbindung mit dem Promotor umfasst.

12. Verwendung nach Anspruch 1 oder 2, wobei die Symptome ausgewählt sind aus der Gruppe, die umfasst: eine anormale Körperfunktion, eine anormale Zellmorphologie, anormale Enzympegel, anormale Hormonpegel, anormale onkofötale Antigenpegel, ein anormales Gewebewachstum, eine anormale Gewebemasse, anormale einem Tumor zugeordnete Proteinpegel, eine veränderte neurologische Funktion, eine veränderte neurologische Struktur, eine Angiogenese, eine Blutung, Zellen, die einen Krebszellenphänotyp besitzen, eine Diarrhö, Blutergüsse, eine Müdigkeit, ein Fieber, Läsionen, eine Unterernährung, eine Metastase, einen Brechreiz, eine Behinderung von einem Körperdurchgang, eine opportunistische Infektion, einen Schmerz, einen schlechten Karnofsky-Leistungszustand, eine Anwesenheit von Markern der Zelloberfläche, eine Anwesenheit von histologischen Markern, eine Anwesenheit von intrazellulären Markern, eine Anwesenheit von molekularen Markern, eine Tumorinvasion, eine unregulierte Zellproliferation, eine Harnhäufigkeit und einen Gewichtsverlust.

13. Verwendung nach Anspruch 1 oder 2, wobei das Morphogen den zellulären Einbau von [³H]-Thymidin in einer *in vitro* Probe inhibiert.

14. Verwendung nach Anspruch 1 oder 2, wobei das Medikament ferner mindestens eine Substanz in Kombination mit dem Morphogen umfasst.

15. Verwendung nach Anspruch 14, wobei die Substanz ein Anti-Krebs-Wirkstoff ist.

16. Verwendung nach Anspruch 1 oder 2, wobei die Krebszellen mindestens einen Rezeptor für ein knochenmorphogenes Protein (BMPR) exprimieren, der spezifisch für das Morphogen ist.

17. Verwendung nach Anspruch 16, wobei das BMPR aus der Gruppe ausgewählt ist, die umfasst BMPR-IA, BMPR-IB, BMPR-II und deren Spleißvarianten.

18. Verwendung nach Anspruch 1 oder 2, wobei das Morphogen die Tumorgröße oder die Tumorentwicklung verringert.

19. Verwendung nach Anspruch 1 oder 2, wobei das Morphogen die Überlebensrate erhöht.

20. Verwendung nach Anspruch 1 oder 2, wobei das Morphogen eine Tumornekrose und eine diffuse Entzündung zur Folge hat.

## Revendications

1. Utilisation d'un morphogène comprenant une protéine dimérique, où le morphogène est sélectionné parmi OP-1 et BMP-6 dans la fabrication d'un médicament pour soulager les symptômes du cancer de la prostate, et où le morphogène empêche la croissance des cellules cancéreuses.

2. Utilisation d'un morphogène comprenant une protéine dimérique, où le morphogène est sélectionné parmi OP-1 dans la fabrication d'un médicament pour soulager les symptômes du cancer du sein, et où le morphogène empêche la croissance des cellules cancéreuses.

3. Utilisation selon la revendication 1, où le morphogène est OP-1.

4. Utilisation selon la revendication 1, où le morphogène est BMP-6.

5. Utilisation selon l'une quelconque des revendications précédentes, où le morphogène est présent dans un véhicule pharmaceutiquement acceptable.

6. Utilisation selon la revendication 5, où le véhicule est sélectionné dans le groupe consistant en microsphères biocompatibles, microsphères biodégradables et microsphères bioérodables.

7. Utilisation selon la revendication 5, où le véhicule est sélectionné dans le groupe consistant en matrices biocompatibles, matrices biodégradables et matrices bioérodables.

8. Utilisation selon la revendication 5, où le véhicule est une solution aqueuse.

9. Utilisation selon la revendication 1 ou 2, où le médicament comprend des cellules aptes à exprimer le morphogène.

10. Utilisation selon la revendication 1 ou 2, où le médicament comprend une ADN non-infectieuse, non-intégrante codant pour le morphogène fonctionnellement lié à un promoteur, où la ADN est exempte d'une association avec des protéines facilitant la transfection, particules virales, formulations liposomales, lipides chargés et agents de précipitation de phosphate de calcium.

11. Utilisation selon la revendication 10, où la ADN non-infectieuse, non-intégrante codant pour le morphogène fonctionnellement lié au promoteur comprend en outre un élément amplificateur fonctionnellement associé au promoteur.

12. Utilisation selon la revendication 1 ou 2, où les symptômes sont sélectionnés dans le groupe consistant en une fonction corporelle anormale, morphologie de cellules anormale, niveaux d'enzymes anormaux, niveaux d'hormones anormaux, niveaux d'antigènes oncofoetaux anormaux, croissance de tissu anormal, masse de tissu anormal, niveaux de protéines anormaux associés à la tumeur, fonction neurologique modifiée, structure neurologique modifiée, angiogénèse, saignement, cellules ayant un phénotype de cellules cancéreuses, diarrhée, épanchements, fatigue, fièvre, lésions, malnutrition, métastase, nausée, obstruction d'un passage corporel, infection opportuniste, douleur, indice de Karnofsky médiocre, présence de marqueurs de surface de cellule, présence de marqueurs histologiques, présence de marqueurs intracellulaires, présence de marqueurs moléculaires, invasion tumorale, prolifération de cellules non régulée, fréquence urinaire et perte de poids.

13. Utilisation selon la revendication 1 ou 2, où le morphogène empêche une incorporation cellulaire de [³H]-thymidine dans une analyse in vitro.

14. Utilisation selon la revendication 1 ou 2, où le médicament comprend en outre au moins une substance en combinaison avec le morphogène.

15. Utilisation selon la revendication 14, où la substance est un agent anti-cancer.

16. Utilisation selon la revendication 1 ou 2, où les cellules cancéreuses expriment au moins un récepteur de protéine morphogénique d'os (BMPR) spécifique pour le morphogène.

17. Utilisation selon la revendication 16, où le BMPR est sélectionné dans le groupe consistant en BMPR-IA, BMPR-IB, BMPR-II, et leurs variantes annexes.

18. Utilisation selon la revendication 1 ou 2, où le morphogène réduit la taille ou le développement de la tumeur.

19. Utilisation selon la revendication 1 ou 2, où le morphogène fait augmenter le taux de survie.

20. Utilisation selon la revendication 1 ou 2, où le morphogène se traduit par une nécrose de la tumeur et une inflammation diffuse.


### SEQUENCE LISTING

<110> Creative Biomolecules, Inc.
   Sampath, et al.
<120> Methods of Alleviating Cancer Symptoms
<130> CRP-166 PCT (00960-521; CBM-21)
<140> filed herewith
   <141> 1999-11-12
<160> 9
<170> Patent In Ver. 2.0
<210> 1
   <211> 1822
   <212> DNA
   <213> Artificial Sequence
<220>
   <221> CDS
   <222> (49)..(1341)
   <223> Osteogenic protein; OP1
<400> 1
   ggtgcgggcc cggagcccgg agcccgggta gcgcgtagag ccggcgcg atg cac gtg  57
                                                        Met His Val
                                                          1
   cgc tca ctg cga gct gcg gcg ccg cac agc ttc gtg gcg ctc tgg gca  105
   Arg Ser Leu Arg Ala Ala Ala Pro His Ser Phe Val Ala Leu Trp Ala
         5                  10                  15
   ccc ctg ttc ctg ctg cgc tcc gcc ctg gcc gac ttc agc ctg gac aac  153
   Pro Leu Phe Leu Leu Arg Ser Ala Leu Ala Asp Phe Ser Leu Asp Asn
    20                  25                  30                  35
   gag gtg cac tcg agc ttc atc cac cgg cgc ctc cgc agc cag gag cgg  201
   Glu Val His Ser Ser Phe Ile His Arg Arg Leu Arg Ser Gln Glu Arg
                    40                  45                  50
   cgg gag atg cag cgc gag atc ctc tcc att ttg ggc ttg ccc cac cgc  249
   Arg Glu Met Gln Arg Glu Ile Leu Ser Ile Leu Gly Leu Pro His Arg
                55                  60                  65
   ccg cgc ccg cac ctc cag ggc aag cac aac tcg gca ccc atg ttc atg  297
   Pro Arg Pro His Leu Gln Gly Lys His Asn Ser Ala Pro Met Phe Met
   70 75 80
   ctg gac ctg tac aac gcc atg gcg gtg gag gag ggc ggc ggg ccc ggc 345
   Leu Asp Leu Tyr Asn Ala Met Ala Val Glu Glu Gly Gly Gly Pro Gly
   85 90 95
   ggc cag ggc ttc tcc tac ccc tac aag gcc gtc ttc agt acc cag ggc 393
   Gly Gln Gly Phe Ser Tyr Pro Tyr Lys Ala Val Phe Ser Thr Gln Gly
   100 105 110 115
   ccc cct ctg gcc agc ctg caa gat agc cat ttc ctc acc gac gcc gac 441
   Pro Pro Leu Ala Ser Leu Gln Asp Ser His Phe Leu Thr Asp Ala Asp
   120 125 130
   atg gtc atg agc ttc gtc aac ctc gtg gaa cat gac aag gaa ttc ttc 489
   Met Val Met Ser Phe Val Asn Leu Val Glu His Asp Lys Glu Phe Phe
   135 140 145
   cac cca cgc tac cac cat cga gag ttc cgg ttt gat ctt tcc aag atc 537
   His Pro Arg Tyr His His Arg Glu Phe Arg Phe Asp Leu Ser Lys Ile
   150 155 160
   cca gaa ggg gaa gct gtc acg gca gcc gaa ttc cgg atc tac aag gac 585
   Pro Glu Gly Glu Ala Val Thr Ala Ala Glu Phe Arg Ile Tyr Lys Asp
   165 170 175
   tac atc cgg gaa cgc ttc gac aat gag acg ttc cgg atc agc gtt tat 633
   Tyr Ile Arg Glu Arg Phe Asp Asn Glu Thr Phe Arg Ile Ser Val Tyr
   180 185 190 195
   cag gtg ctc cag gag cac ttg ggc agg gaa tcg gat ctc ttc ctg ctc 681
   Gln Val Leu Gln Glu His Leu Gly Arg Glu Ser Asp Leu Phe Leu Leu
   200 205 210
   gac agc cgt acc ctc tgg gcc tcg gag gag ggc tgg ctg gtg ttt gac 729
   Asp Ser Arg Thr Leu Trp Ala Ser Glu Glu Gly Trp Leu Val Phe Asp
   215 220 225
   atc aca gcc acc agc aac cac tgg gtg gtc aat ccg cgg cac aac ctg 777
   Ile Thr Ala Thr Ser Asn His Trp Val Val Asn Pro Arg His Asn Leu
   230 235 240
   ggc ctg cag ctc tcg gtg gag acg ctg gat ggg cag agc atc aac ccc 825
   Gly Leu Gln Leu Ser Val Glu Thr Leu Asp Gly Gln Ser Ile Asn Pro
   245 250 255
   aag ttg gcg ggc ctg att ggg cgg cac ggg ccc cag aac aag cag ccc 873
   Lys Leu Ala Gly Leu Ile Gly Arg His Gly Pro Gln Asn Lys Gln Pro
   260                 265                 270                 275
   ttc atg gtg gct ttc ttc aag gcc acg gag gtc cac ttc cgc agc atc     921
   Phe Met Val Ala Phe Phe Lys Ala Thr Glu Val His Phe Arg Ser Ile
                   280                 285                 290
   cgg tcc acg ggg agc aaa cag cgc agc cag aac cgc tcc aag acg ccc     969
   Arg Ser Thr Gly Ser Lys Gln Arg Ser Gln Asn Arg Ser Lys Thr Pro
               295                 300                 305
   aag aac cag gaa gcc ctg cgg atg gcc aac gtg gca gag aac agc agc    1017
   Lys Asn Gln Glu Ala Leu Arg Met Ala Asn Val Ala Glu Asn Ser Ser
           310                 315                 320
   agc gac cag agg cag gcc tgt aag aag cac gag ctg tat gtc agc ttc    1065
   Ser Asp Gln Arg Gln Ala Cys Lys Lys His Glu Leu Tyr Val Ser Phe
       325                 330                 335
   cga gac ctg ggc tgg cag gac tgg atc atc gcg cct gaa ggc tac gcc    1113
   Arg Asp Leu Gly Trp Gln Asp Trp Ile Ile Ala Pro Glu Gly Tyr Ala
   340                 345                 350                 355
   gcc tac tac tgt gag ggg gag tgt gcc ttc cct ctg aac tcc tac atg    1161
   Ala Tyr Tyr Cys Glu Gly Glu Cys Ala Phe Pro Leu Asn Ser Tyr Met
                   360                 365                 370
   aac gcc acc aac cac gcc atc gtg cag acg ctg gtc cac ttc atc aac    1209
   Asn Ala Thr Asn His Ala Ile Val Gln Thr Leu Val His Phe Ile Asn
               375                 380                 385
   ccg gaa acg gtg ccc aag ccc tgc tgt gcg ccc acg cag ctc aat gcc    1257
   Pro Glu Thr Val Pro Lys Pro Cys Cys Ala Pro Thr Gln Leu Asn Ala
           390                 395                 400
   atc tcc gtc ctc tac ttc gat gac agc tcc aac gtc atc ctg aag aaa    1305
   Ile Ser Val Leu Tyr Phe Asp Asp Ser Ser Asn Val Ile Leu Lys Lys
       405                 410                 415
   tac aga aac atg gtg gtc cgg gcc tgt ggc tgc cac tagctcctcc         1351
   Tyr Arg Asn Met Val Val Arg Ala Cys Gly Cys His
   420                 425                 430
   gagaattcag accctttggg gccaagtttt tctggatcct ccattgctcg ccttggccag 1411
   gaaccagcag accaactgcc ttttgtgaga ccttcccctc cctatcccca actttaaagg 1471
   tgtgagagta ttaggaaaca tgagcagcat atggcttttg atcagttttt cagtggcagc 1531
   atccaatgaa caagatccta caagctgtgc aggcaaaacc tagcaggaaa aaaaaacaac 1591
   gcataaagaa aaatggccgg gccaggtcat tggctgggaa gtctcagcca tgcacggact 1651
   cgtttccaga ggtaattatg agcgcctacc agccaggcca cccagccgtg ggaggaaggg 1711
   ggcgtggcaa ggggtgggca cattggtgtc tgtgcgaaag gaaaattgac ccggaagttc 1771
   ctgtaataaa tgtcacaata aaacgaatga atgaaaaaaa aaaaaaaaaa a 1822
<210> 2
   <211> 431
   <212> PRT
   <213> Artificial Sequence
<400> 2
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
   115 120 125
   Asp Ala Asp Met Val Met Ser Phe Val Asn Leu Val Glu His Asp Lys
   130 135 140
   Glu Phe Phe His Pro Arg Tyr His His Arg Glu Phe Arg Phe Asp Leu
   145 150 155 160
   Ser Lys Ile Pro Glu Gly Glu Ala Val Thr Ala Ala Glu Phe Arg Ile
   165 170 175
   Tyr Lys Asp Tyr Ile Arg Glu Arg Phe Asp Asn Glu Thr Phe Arg Ile
   180 185 190
   Ser Val Tyr Gln Val Leu Gln Glu His Leu Gly Arg Glu Ser Asp Leu
   195 200 205
   Phe Leu Leu Asp Ser Arg Thr Leu Trp Ala Ser Glu Glu Gly Trp Leu
   210 215 220
   Val Phe Asp Ile Thr Ala Thr Ser Asn His Trp Val Val Asn Pro Arg
   225 230 235 240
   His Asn Leu Gly Leu Gln Leu Ser Val Glu Thr Leu Asp Gly Gln Ser
   245 250 255
   Ile Asn Pro Lys Leu Ala Gly Leu Ile Gly Arg His Gly Pro Gln Asn
   260 265 270
   Lys Gln Pro Phe Met Val Ala Phe Phe Lys Ala Thr Glu Val His Phe
   275 280 285
   Arg Ser Ile Arg Ser Thr Gly Ser Lys Gln Arg Ser Gln Asn Arg Ser
   290 295 300
   Lys Thr Pro Lys Asn Gln Glu Ala Leu Arg Met Ala Asn Val Ala Glu
   305 310 315 320
   Asn Ser Ser Ser Asp Gln Arg Gln Ala Cys Lys Lys His Glu Leu Tyr
   325 330 335
   Val Ser Phe Arg Asp Leu Gly Trp Gln Asp Trp Ile Ile Ala Pro Glu
   340 345 350
   Gly Tyr Ala Ala Tyr Tyr Cys Glu Gly Glu Cys Ala Phe Pro Leu Asn
   355 360 365
   Ser Tyr Met Asn Ala Thr Asn His Ala Ile Val Gln Thr Leu Val His
   370 375 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Arg Asn Met Val Val Arg Ala Cys Gly Cys His
   420 425 430
<210> 3
   <211> 102
   <212> PRT
   <213> Artificial Sequence
<220>
   <221> PEPTIDE
   <222> (1)..(102)
   <223> OPX; where each Xaa is independently selected from a group of one or more specified amino acids as defined in the specification
<400> 3
   Cys Xaa Xaa His Glu Leu Tyr Val Ser Phe Xaa Asp Leu Gly Trp Xaa
   1 5 10 15
   Asp Trp Xaa Ile Ala Pro Xaa Gly Tyr Xaa Ala Tyr Tyr Cys Glu Gly
   20 25 30
   Glu Cys Xaa Phe Pro Leu Xaa Ser Xaa Met Asn Ala Thr Asn His Ala
   35 40 45
   Ile Xaa Gln Xaa Leu Val His Xaa Xaa Xaa Pro Xaa Xaa Val Pro Lys
   50 55 60
   Xaa Cys Cys Ala Pro Thr Xaa Leu Xaa Ala Xaa Ser Val Leu Tyr Xaa
   65 70 75 80
   Asp Xaa Ser Xaa Asn Val Ile Leu Xaa Lys Xaa Arg Asn Met Val Val
   85 90 95
   Xaa Ala Cys Gly Cys His
   100
<210> 4
   <211> 97
   <212> PRT
   <213> Artificial Sequence
<220>
   <221> PEPTIDE
   <222> (1)..(97)
   <223> Generic-Seq-7; wherein each xaa is independently selected from a group of one or more specified amino acids as defined in the specification
<400> 4
   Leu Xaa Xaa Xaa Phe Xaa Xaa Xaa Gly Trp Xaa Xaa Xaa Xaa Xaa Xaa
   1 5 10 15
   Pro Xaa Xaa Xaa Xaa Ala Xaa Tyr Cys Xaa Gly Xaa Cys Xaa Xaa Pro
   20 25 30
   Xaa Xaa Xaa Xaa Xaa Xaa Xaa Xaa Asn His Ala Xaa Xaa Xaa Xaa Xaa
   35 40 45
   Xaa Xaa Xaa Xaa Xaa Xaa Xaa Xaa Xaa Xaa Xaa Xaa Cys Cys Xaa Pro
   50 55 60
   Xaa Xaa Xaa Xaa Xaa Xaa Xaa Xaa Leu Xaa Xaa Xaa Xaa Xaa Xaa Xaa
   65 70 75 80
   Val Xaa Leu Xaa Xaa Xaa Xaa Xaa Met Xaa Val Xaa Xaa Cys Xaa Cys
   85 90 95
   Xaa
<210> 5
   <211> 102
   <212> PRT
   <213> Artificial Sequence
<220>
   <221> PEPTIDE
   <222> (1)..(102)
   <223> Generic-Seq-8; wherein each Xaa is independently selected from a group of one or more specified amino acids as defined in the specification
<400> 5
   Cys Xaa Xaa Xaa Xaa Leu Xaa Xaa Xaa Phe Xaa Xaa Xaa Gly Trp Xaa
   1 5 10 15
   Xaa Xaa Xaa Xaa Xaa Pro Xaa Xaa Xaa Xaa Ala Xaa Tyr Cys Xaa Gly
   20 25 30
   Xaa Cys Xaa Xaa Pro Xaa Xaa Xaa Xaa Xaa Xaa Xaa Xaa Asn His Ala
   35 40 45
   Xaa Xaa Xaa Xaa Xaa Xaa Xaa Xaa Xaa Xaa Xaa Xaa Xaa Xaa Xaa Xaa
   50 55 60
   Xaa Cys Cys Xaa Pro Xaa Xaa Xaa Xaa Xaa Xaa Xaa Xaa Leu Xaa Xaa
   65 70 75 80
   Xaa Xaa Xaa Xaa Xaa Val Xaa Leu Xaa Xaa Xaa Xaa Xaa Met Xaa Val
   85 90 95
   Xaa Xaa Cys Xaa Cys Xaa
   100
<210> 6
   <211> 97
   <212> PRT
   <213> Artificial Sequence
<220>
   <221> PEPTIDE
   <222> (1)..(97)
   <223> Generic-Seq-9; wherein each Xaa is independently selected from a group of one or more specified amino acids as defined in the specification
<400> 6
   Xaa Xaa Xaa Xaa Xaa Xaa Xaa Xaa Xaa Xaa Xaa Xaa Xaa Xaa Xaa Xaa
   1 5 10 15
   Pro Xaa Xaa Xaa Xaa Xaa Xaa Xaa Cys Xaa Gly Xaa Cys Xaa Xaa Xaa
   20 25 30
   Xaa Xaa Xaa Xaa Xaa Xaa Xaa Xaa Xaa Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Cys Xaa Pro
   50 55 60
   Xaa Xaa Xaa Xaa Xaa Xaa Xaa Xaa Leu Xaa Xaa Xaa Xaa Xaa Xaa Xaa
   65 70 75 80
   Xaa Xaa Xaa Xaa Xaa Xaa Xaa Xaa Xaa Xaa Xaa Xaa Xaa Cys Xaa Cys
   85 90 95
   Xaa
<210> 7
   <211> 102
   <212> PRT
   <213> Artificial Sequence
<220>
   <221> PEPTIDE
   <222> (1)..(102)
   <223> Generic-Seq-10; wherein each Xaa is independently selected from a group of one or more specified amino acids as defined in the specification
<400> 7
   Cys Xaa Xaa Xaa Xaa Xaa Xaa Xaa Xaa Xaa Xaa Xaa Xaa Xaa Xaa Xaa
   1 5 10 15
   Xaa Xaa Xaa Xaa Xaa Pro Xaa Xaa Xaa Xaa Xaa Xaa Xaa Cys Xaa Gly
   20 25 30
   Xaa Cys Xaa Xaa Xaa Xaa Xaa Xaa Xaa Xaa Xaa Xaa Xaa Xaa Xaa Xaa
   35 40 45
   Xaa Xaa Xaa Xaa Xaa Xaa Xaa Xaa Xaa Xaa Xaa Xaa Xaa Xaa Xaa Xaa
   50 55 60
   Xaa Xaa Cys Xaa Pro Xaa Xaa Xaa Xaa Xaa Xaa Xaa Xaa Leu Xaa Xaa
   65 70 75 80
   Xaa Xaa Xaa Xaa Xaa Xaa Xaa Xaa Xaa Xaa Xaa Xaa Xaa Xaa Xaa Xaa
   85 90 95
   Xaa Xaa Cys Xaa Cys Xaa
   100
<210> 8
   <211> 5
   <212> PRT
   <213> Artificial Sequence
<220>
   <221> PEPTIDE
   <222> (1)..(5)
   <223> wherein each Xaa is independently selected from a group of one or more specified amino acids as defined in the specification
<400> 8
   Cys Xaa Xaa Xaa Xaa
   1 5
<210> 9
   <211> 5
   <212> PRT
   <213> Artificial Sequence
<220>
   <221> PEPTIDE
   <222> (1)..(5)
   <223> wherein each Xaa is independently selected from a group of one or more specified amino acids as defined in the specification
<400> 9
   Cys Xaa Xaa Xaa Xaa
   1 5